(19) Europäisches Patentamt
European Patent Office
Office européen des brevets

(11) **EP 3 873 504 B1**

(12) **EUROPEAN PATENT SPECIFICATION**

(45) Date of publication and mention
of the grant of the patent:
**13.12.2023 Bulletin 2023/50**

(51) International Patent Classification (IPC):
**A61K 35/35** (2015.01)     **G01N 33/574** (2006.01)
**C12N 5/00** (2006.01)      **A61K 9/51** (2006.01)
**A61P 35/00** (2006.01)     **C12N 5/077** (2010.01)
**A61K 47/54** (2017.01)

(21) Application number: **19880619.2**

(22) Date of filing: **01.11.2019**

(52) Cooperative Patent Classification (CPC):
**A61K 47/542; A61K 9/5176; A61K 35/35;
A61P 35/00; C12N 5/0012; C12N 5/0653;**
C12N 2500/36; C12N 2502/1305; C12N 2502/30;
C12N 2510/00

(86) International application number:
**PCT/US2019/059370**

(87) International publication number:
**WO 2020/092887 (07.05.2020 Gazette 2020/19)**

(54) **ADIPOCYTE MEDIATED DELIVERY OF ANTICANCER THERAPEUTICS**

ADIPOZYTENVERMITTELTE VERABREICHUNG VON ANTIKREBSTHERAPEUTIKA

APPORT D'AGENTS THÉRAPEUTIQUES ANTICANCÉREUX MÉDIÉ PAR DES ADIPOCYTES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.11.2018 US 201862754280 P**

(43) Date of publication of application:
**08.09.2021 Bulletin 2021/36**

(73) Proprietor: **North Carolina State University
Raleigh, North Carolina 27606 (US)**

(72) Inventors:
• **GU, Zhen**
  **San Diego,**
  **CA 92130 (US)**
• **WEN, Di**
  **Raleigh, North Carolina 27606 (US)**
• **ZHANG, Xudong**
  **Raleigh, North Carolina 27606 (US)**

(74) Representative: **Berggren Oy
P.O. Box 16
Eteläinen Rautatiekatu 10A
00101 Helsinki (FI)**

(56) References cited:
**US-A1- 2012 289 570      US-A1- 2017 168 041
US-A1- 2018 196 034**

• **TIAN YANHUA ET AL: "A doxorubicin delivery
platform using engineered natural membrane
vesicle exosomes for targeted tumor therapy",
BIOMATERIALS, ELSEVIER, AMSTERDAM, NL,
vol. 35, no. 7, 15 December 2013 (2013-12-15),
pages 2383-2390, XP028807713, ISSN: 0142-9612,
DOI: 10.1016/J.BIOMATERIALS.2013.11.083**
• **CHUNXI LIU ET AL: "The targeted co-delivery of
DNA and doxorubicin to tumor cells via
multifunctional PEI-PEG based nanoparticles",
BIOMATERIALS, vol. 34, no. 10, 1 March 2013
(2013-03-01) , pages 2547-2564, XP055076473,
ISSN: 0142-9612, DOI:
10.1016/j.biomaterials.2012.12.038**
• **SENAPATI SUDIPTA ET AL: "Controlled drug
delivery vehicles for cancer treatment and their
performance", SIGNAL TRANSDUCTION AND
TARGETED THERAPY, vol. 3, no. 1, 16 March 2018
(2018-03-16), XP055937001, DOI:
10.1038/s41392-017-0004-3 Retrieved from the
Internet:
URL:http://www.nature.com/articles/s41392-
017-0004-3>**

- YE MINGZHOU ET AL: "Drug Loaded Adipocytes: Sugar-Coated Bullets for Cancer", MATTER, vol. 1, no. 5, 1 November 2019 (2019-11-01), pages 1104-1105, XP055936331, US ISSN: 2590-2385, DOI: 10.1016/j.matt.2019.09.023
- DI WEN ET AL: "Adipocytes as Anticancer Drug Delivery Depot", MATTER, vol. 1, no. 5, 1 November 2019 (2019-11-01), pages 1203-1214, XP055729215, US ISSN: 2590-2385, DOI: 10.1016/j.matt.2019.08.007
- LUTZ HALLE ET AL: "Cells and cell derivatives as drug carriers for targeted delivery", MEDICINE IN DRUG DISCOVERY, vol. 3, 1 September 2019 (2019-09-01), page 100014, XP055936328, ISSN: 2590-0986, DOI: 10.1016/j.medidd.2020.100014
- TAO et al.: "ROS-responsive drug delivery systems for biomedical applications", Asian Journal of Pharmaceutical Sciences, vol. 13, no. 2 March 2018 (2018-03), XP055729213, Retrieved from the Internet: URL:https://www.sciencedirect.com/science/article/pii/S1818087617308127 [retrieved on 2019-12-26]
- WEN et al.: Adipocytes as Anticancer Drug Delivery Depot, Matter, vol. 1, no. 5 25 September 2019 (2019-09-25), XP055729215, Retrieved from the Internet: URL:https://www.cell.com/matter/fulltext/S 2590-2385(19)30165-1?_returnURL=https%3A% 2 F%2Flinkinghub.elsevier.com%2Fretrieve%2Fp ii%2FS2590238519301651%3Fshowall%3Dtrue [retrieved on 2019-12-17]

## Description

This application claims the benefit of U.S. Provisional Application No. 62/754,280, filed on November 1, 2018.

## I. BACKGROUND

1. Cancer cells generate a supportive microenvironment by recruiting non-malignant cells for tumor development. Recently, tumor associated adipocytes (TAAs) have been considered as endocrine and inflammatory cells, promoting angiogenesis by secreting adipokines, including hormones, growth factors, and cytokines. These adipokines lead to lymphocytes and macrophages recruitment and infiltration in tumor, therefore establishing low grade chronic inflammation. In this tumor microenvironment, fatty acids in lipid droplet of adipocyte can provide energy to cancer cells through fatty acid-binding protein 4 (FABP4) caused by increased lipolysis in the tumor tissue. Furthermore, the peri-tumoral adipose tissue facilitates to recruit tumor associated macrophages (TAM) derived from circulating monocytes, followed by inducing a shift of TAM to an M2 phenotype. Hence, adipocytes represent high potential for regulating tumor growth with high compatibility to the tumor microenvironment. What are needed are new therapeutics that can target adipocyte microenvironment and use the cancer tissue triggering of lipolysis to provide the therapeutics release.

## II. SUMMARY

2. Disclosed are methods and compositions related to compositions and methods related to the use of adipocytes for sustained release of anti-cancer therapeutics and treatment of cancer.

3. In one aspect, disclosed herein are engineered adipocytes comprising an anti-cancer prodrug (such as, for example, doxorubicin prodrug) and a conjugated fatty acid (such as, for example, one or more isomers of conjugated linoleic acid including, but not limited, to 9cis, 11trans, 10trans, and/or 12cis). In one aspect, the prodrug can be conjugated to the conjugated fatty acid via an environmentally reactive linker (such as, for example, a pH sensitive, enzymatic, and/or reactive oxygen species responsive linker). In one aspect, the conjugated fatty acid comprises rumenic acid (9cis, 11 trans linoleic acid). Thus, in one aspect, are engineered adipocytes of any preceding aspect, wherein the anti-cancer prodrug comprises doxorubicin prodrug and rumenic acid.

4. Also disclosed herein are adipocytes of any preceding aspect further comprising a lipid transport protein (such as, for example) fatty-acid binding protein 4 (FABP4).

5. In one aspect, disclosed herein are engineered adipocytes for use in treating, inhibiting, reducing, and/or preventing a cancer or metastasis in a subject

6. Also disclosed herein are methods of providing sustained release of an anti-cancer agent to a tumor comprising conjugating the anti-cancer agent to a conjugated fatty acid, encapsulating the conjugated anti-cancer agent in an adipocyte to make an engineered adipocyte, and delivering the engineered adipocyte to a tumor.

## III. BRIEF DESCRIPTION OF THE DRAWINGS

7. The accompanying drawings, which are incorporated in and constitute a part of this specification, illustrate several embodiments and together with the description illustrate the disclosed compositions and methods.

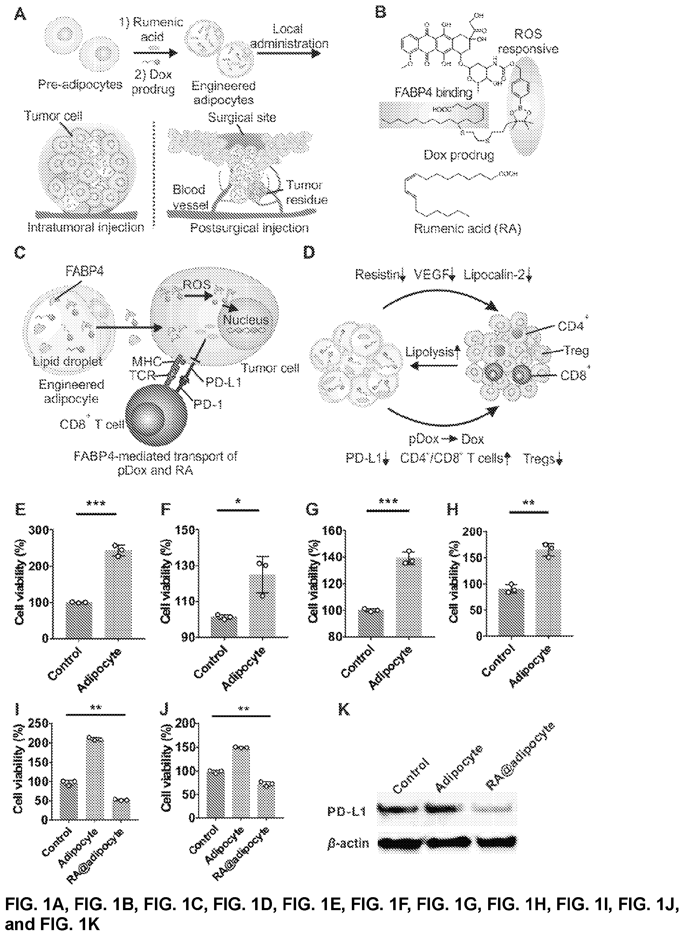

8. Figures 1A, 1B, 1C, 1D, 1E, 1F, 1G, 1H, 1I, 1J, and 1K show RA reversed the malignant role of adipocyte. Scheme of the overall project. Figure 1A shows that pDox and RA were encapsulated into adipocytes and further intratumorally or postsurgically injected. Figure 1B shows the structure of Dox prodrug and rumenic acid. Figure 1C shows the crosstalk between pDox+RA@adipocytes and tumor cells. Figure 1D shows the therapeutic effect of pDox+RA@adipocyte. Figures 1E, 1F, 1G, and 1H show that normal adipocyte can promote tumor cell growth in a transwell system, including B16F10 (1E), A375 (1F), E0771 (1G), and MCF-7 (1H). Figures 1I and IJ shows that when RA or CLA were added during differentiation, new adipocyte can suppress B16F10 (1I) and E0771 (1J) cell growth. Figure 1K shows PD-L1 expression of B16F10 in the same transwell system were determined by Western blot. All bars represent means $\pm$ s.d. (n=3). Unpaired student t test was performed. *P < 0.05, **P < 0.01, ***P < 0.001.

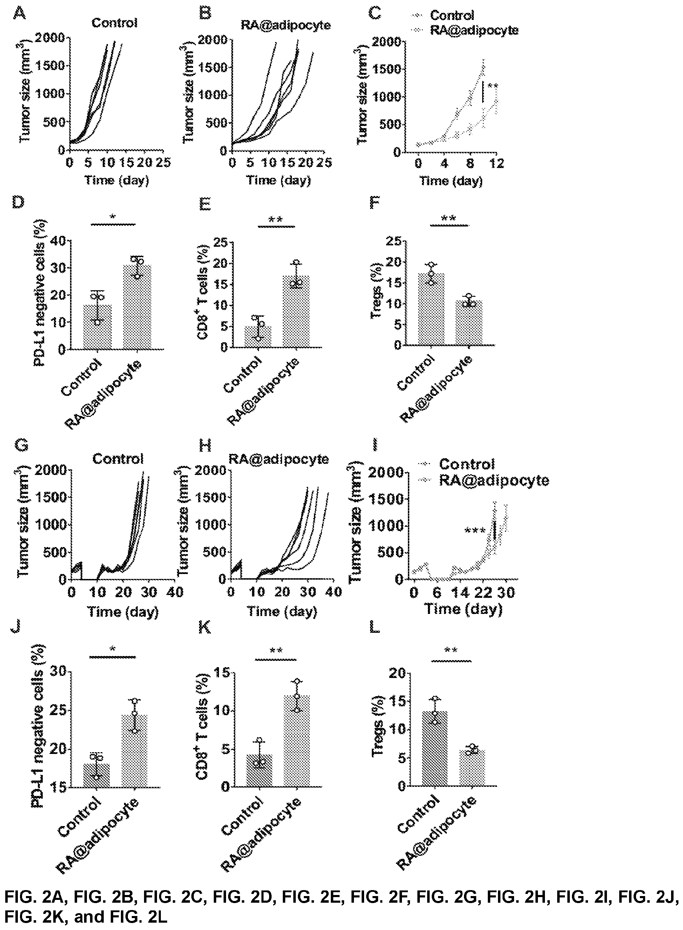

9. Figures 2A, 2B, 2C, 2D, 2E, 2F, 2G, 2H, 2I, 2J, 2K, and 2L show RA inhibited tumor growth and postsurgical recurrence of B 16F10 tumors. Figures 2A, 2B, and 2C show tumor growth after intratumorally injection of RA@adipocyte was monitored as shown by individual (control (2A) and RA@adipocyte (2B)) and average (2C) tumor growth kinetics in control and treated groups. Figures 2D, 2E, and 2F show PD-L1 expression (2D), the population of CD8 T cells (2E) and Tregs (2F) were determined by flow cytometry. Figures 2G, 2H, and 2I show postsurgical tumor growth was indicated by individual (control (2G) and RA@adipocyte (2H)) and average (2I) tumor growth kinetics. Figures 2J, 2K, and 2L show PD-L1 expression (2J), the population of CD8 T cells (2K) and Tregs (2L) were determined by flow cytometry.; 2c, 2i, Bars represent means $\pm$ s.e.m. (n=6). Two-way ANOVA analyses were carried out to do the analyses. 2d, 2e, 2f, 2j, 2k, 2l, Bars represent means $\pm$ s.d. (n=3). Unpaired student t test was performed.

*P < 0.05, **P < 0.01, ***P < 0.001

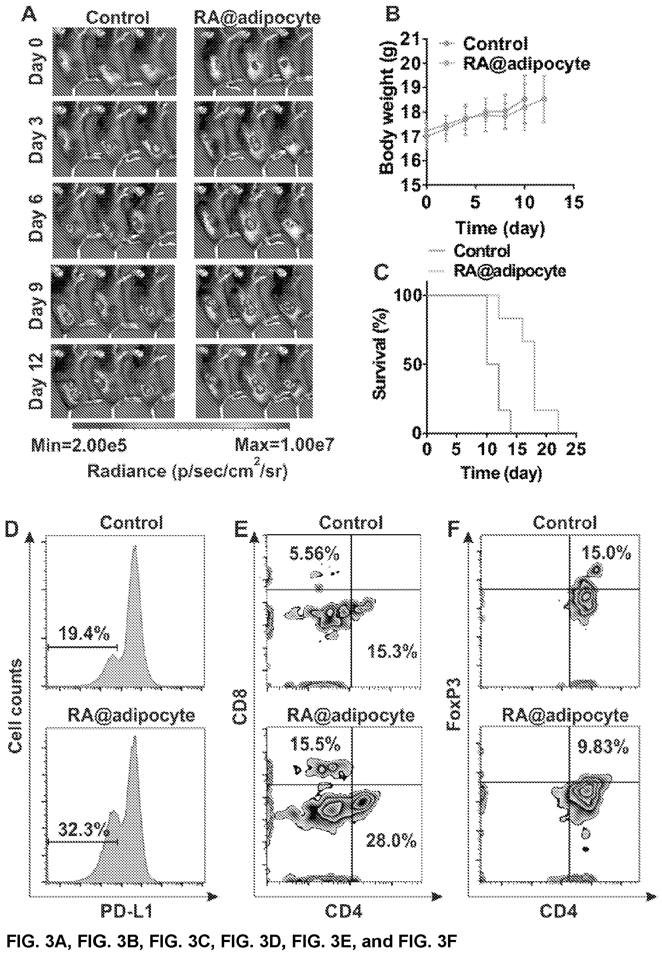

10. Figures 3A, 3B, 3C, 3D, 3E, and 3F show the anti-tumor effect of RA@adiposcyte in an intratumoral model. Figure 3A shows in vivo bioluminescence imaging of the B 16F10 tumor in control and RA@adipocyte treated groups. Figure 3B shows body weight of control and RA@adipocyte treated groups. Figure 3C shows the survival curve of control and RA@adipocyte treated groups. Representative figures of flow cytometry for PD-L1 negative cells (3D), CD8 T cells (3E), and Tregs (3F).

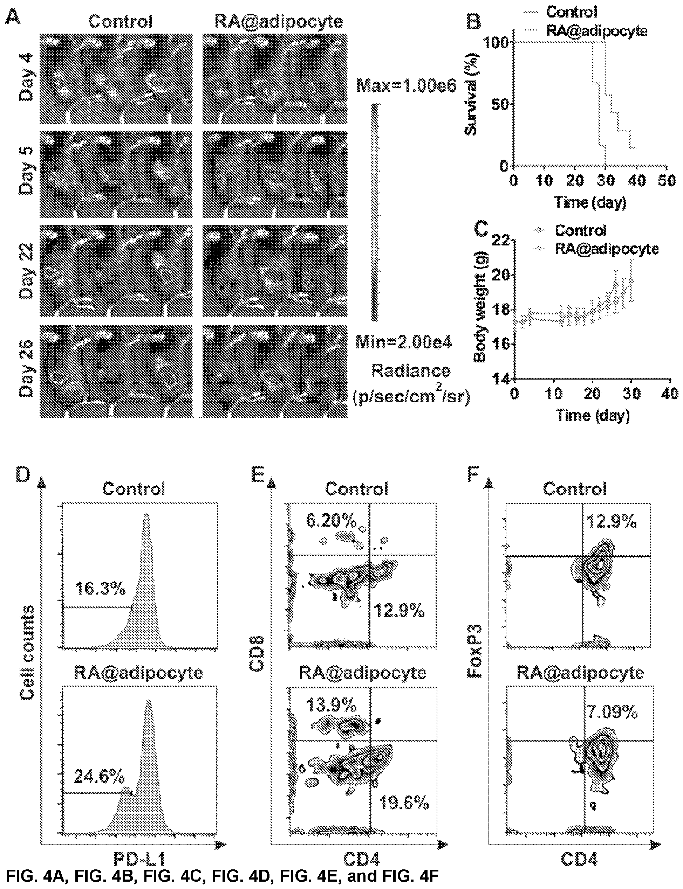

11. Figures 4A, 4B, 4C, 4D, 4E, and 4F show (4A) in vivo bioluminescence imaging of the B16F10 tumor in control and RA@adipocyte treated groups. Figure 4B shows the survival curve of control and RA@adipocyte treated groups. Figure 4C shows body weight of control and RA@adipocyte treated groups. Representative figures of flow cytometry for PD-L1 negative cells (4D), CD8 T cells (4E), and Tregs (4F).

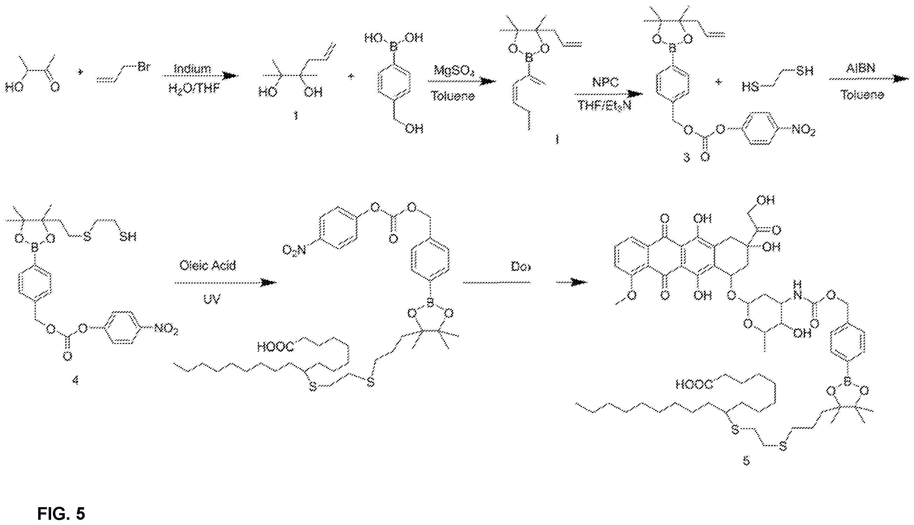

12. Figure 5 shows the synthesis of doxorubicin prodrug.

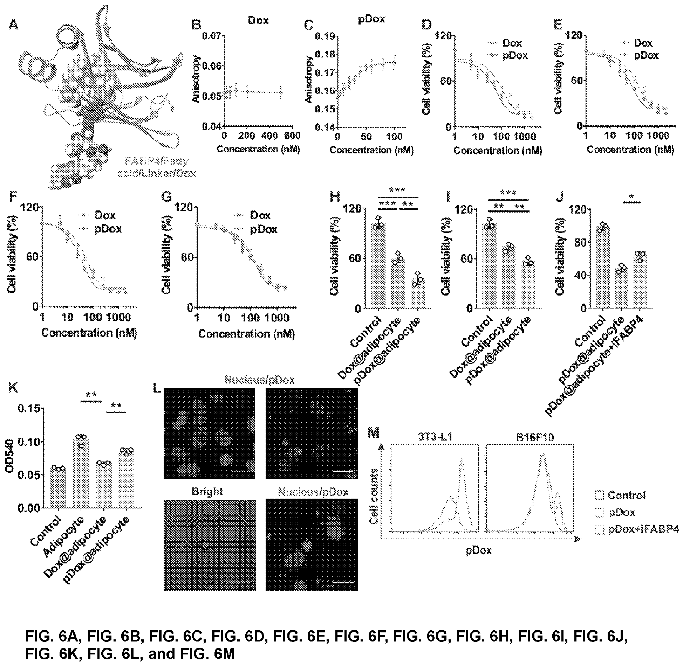

13. Figures 6A, 6B, 6C, 6D, 6E, 6F, 6G, 6H, 6I, 6J, 6K, 6L, and 6M show the characterization of doxorubicin prodrug for adipocyte-based delivery system. a, simulation of pDox and FABP4 binding, b, c, Binding affinity of Dox (b) and pDox (c) were determined by fluorescence polarization. d-g, Cytotoxicity of pDox compared with Dox were determined in B16F10 (d), A375 (e), E0771 (f), and MCF-7 (g) cell lines, h-j, pDox and Dox were further encapsulated into adipocytes and anti-cancer effect of these drug loaded adipocytes were evaluated in B 16F10 (h) and E0771 (i) cell lines, while the effect of FABP4 inhibitor on pDox was evaluated using B 16F10 cell line (j). k, The inhibition effect of Dox and pDox on lipid accumulation was determined by oil red staining. 1, Localization of pDox was determined by fluorescent microscope (Scale bar: 20 μM). m, Uptake efficacy of pDox in cancer cell were determined by flow cytometry after co-culturing B16F10 cells and pDox@adipocytes in a transwell system. All bars represent means ± s.d (n=3). Unpaired student t test was performed. *P < 0.05, **P < 0.01, ***P < 0.001.

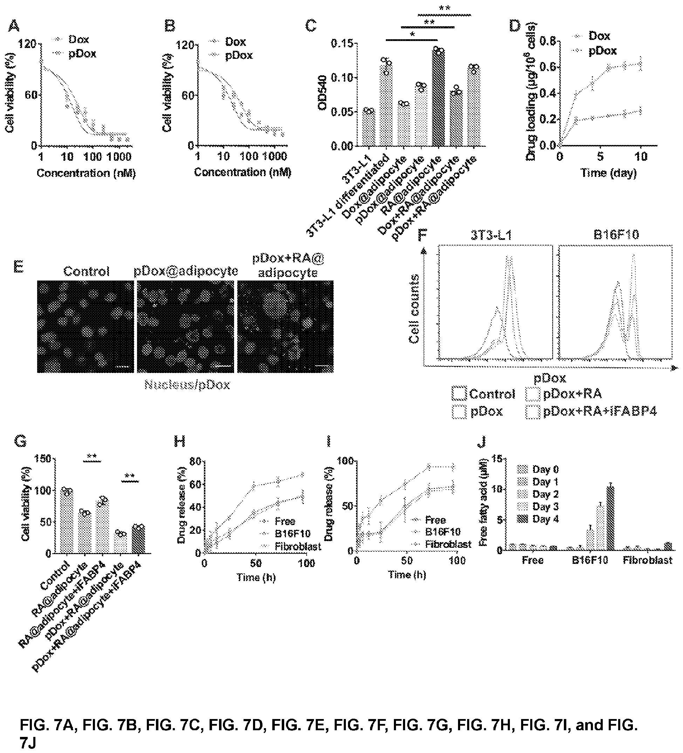

14. Figures 7A, 7B, 7C, 7D, 7E, 7F, 7G, 7H, 7I, and 7J show Combination effect of RA and pDox for cancer therapy. a,b, Anti-cancer effect of RA and Dox or pDox combination therapy was determined in B16F10 (a) and E0771 (b) cell lines, c, Lipid accumulation in adipocytes were evaluated by oil red staining. d, Loading capacity of Dox and pDox in RA@adipocytes was compared. e, RA enhanced the loading capacity determined under confocal fluorescent microscope (Scale bar: 20 μM). f, Crosstalk of B16F10 and Adipocyte in the transwell system through FABP4 was determined by flow cytometry. g, Cytotoxicity of pDox+RA@adipocytes in a transwell system was determined by MTT assay. h-j Release profile of Dox (h) and pDox (i) from adipocytes and the concentration of free fatty acid (j) was determined in a transwell system. All bars represent means ± s.d. (n=3). c, g, unpaired student t test was performed. *P < 0.05, **P < 0.01.

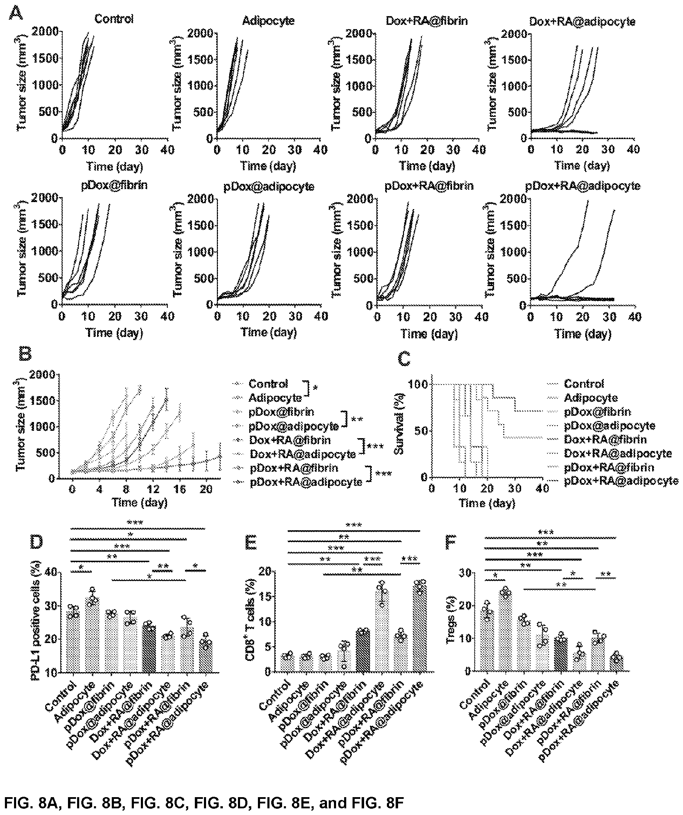

15. Figures 8A, 8B, 8C, 8D, 8E, and 8F show local drug loaded adipocyte suppressed tumor growth. a, Individual tumor growth kinetics. b, Average tumor size in each group. c, Survival curves for different treatment. d-f, Population of PD-L1 positive cells (d), CD8 cells (e) and Tregs (f) was quantified by flow cytometry; b, Bars represent means ± s.e.m. (n=6-7). Two-way ANOVA analyses were carried out to do the analyses. d-f, Bars represent means ± s.d. (n=4). Unpaired student t test was performed. *P < 0.05, **P < 0.01, ***P < 0.001.

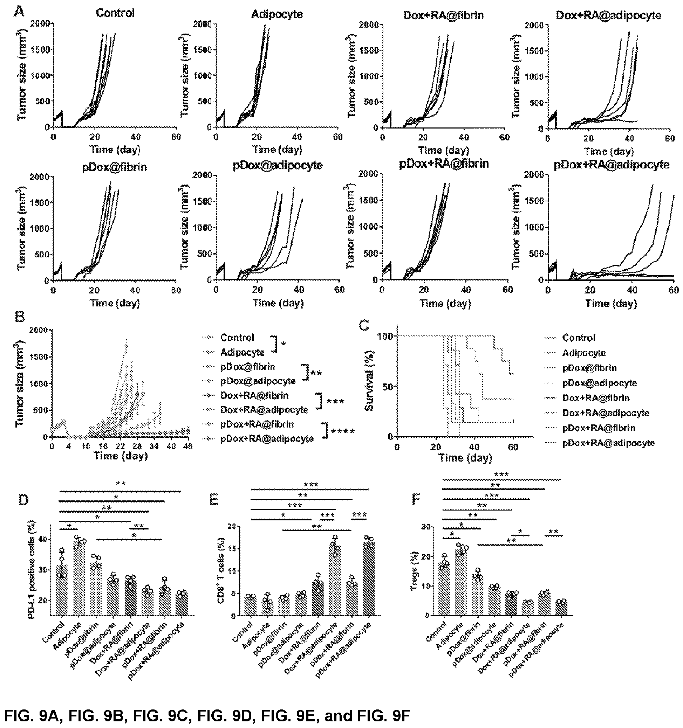

16. Figures 9A, 9B, 9C, 9D, 9E, and 9F show drug loaded adipocyte for inhibition of tumor recurrence of B16F10 tumors. a, Individual tumor growth kinetics. b, Average tumor size in each group. c, Survival curves for different treatment. d-f, Population of CD8 cells (d), PD-L1 positive cells (e) and Tregs (f) was quantified by flow cytometry. b, Bars represent means ± s.e.m. (n=6-8). Two-way ANOVA analyses were carried out to do the analyses. d-f, Bars represent means ± s.d. (n=4). Unpaired student t test was performed. *P < 0.05, **P < 0.01, ***P < 0.001, ****P < 0.0001.

## IV. DETAILED DESCRIPTION

17. Before the present compounds, compositions, articles, devices, and/or methods are disclosed and described, it is to be understood that they are not limited to specific synthetic methods or specific recombinant biotechnology methods unless otherwise specified, or to particular reagents unless otherwise specified, as such may, of course, vary.

### A. Definitions

18. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

19. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

20. In this specification and in the claims which follow, reference will be made to a number of terms which shall be defined to have the following meanings:

21. "Optional" or "optionally" means that the subsequently described event or circumstance may or may not occur, and that the description includes instances where said event or circumstance occurs and instances where it does not.

22. Administration" to a subject includes any route of introducing or delivering to a subject an agent. Administration can be carried out by any suitable route, including oral, topical, intravenous, subcutaneous, transcutaneous, transdermal, intramuscular, intra-joint, parenteral, intra-arteriole, intradermal, intraventricular, intracranial, intraperitoneal, intralesional, intranasal, rectal, vaginal, by inhalation, via an implanted reservoir, parenteral (e.g., subcutaneous, intravenous, intramuscular, intra-articular, intra-synovial, intrasternal, intrathecal, intraperitoneal, intrahepatic, intralesional, and intracranial injections or infusion techniques), and the like. "Concurrent administration", "administration in combination", "simultaneous administration" or "administered simultaneously" as used herein, means that the compounds are administered at the same point in time or essentially immediately following one another. In the latter case, the two compounds are administered at times sufficiently close that the results observed are indistinguishable from those achieved when the compounds are administered at the same point in time. "Systemic administration" refers to the introducing or delivering to a subject an agent via a route which introduces or delivers the agent to extensive areas of the subject's body (e.g. greater than 50% of the body), for example through entrance into the circulatory or lymph systems. By contrast, "local administration" refers to the introducing or delivery to a subject an agent via a route which introduces or delivers the agent to the area or area immediately adjacent to the point of administration and does not introduce the agent systemically in a therapeutically significant amount. For example, locally administered agents are easily detectable in the local vicinity of the point of administration but are undetectable or detectable at negligible amounts in distal parts of the subject's body. Administration includes self-administration and the administration by another.

23. "Biocompatible" generally refers to a material and any metabolites or degradation products thereof that are generally non-toxic to the recipient and do not cause significant adverse effects to the subject.

24. "Comprising" is intended to mean that the compositions, methods, etc. include the recited elements, but do not exclude others. "Consisting essentially of' when used to define compositions and methods, shall mean including the recited elements, but excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of' shall mean excluding more than trace elements of other ingredients and substantial method steps for administering the compositions of this invention. Embodiments defined by each of these transition terms are within the scope of this invention.

25. A "control" is an alternative subject or sample used in an experiment for comparison purposes. A control can be "positive" or "negative."

26. "Controlled release" or "sustained release" refers to release of an agent from a given dosage form in a controlled fashion in order to achieve the desired pharmacokinetic profile in vivo. An aspect of "controlled release" agent delivery is the ability to manipulate the formulation and/or dosage form in order to establish the desired kinetics of agent release.

27. "Effective amount" of an agent refers to a sufficient amount of an agent to provide a desired effect. The amount of agent that is "effective" will vary from subject to subject, depending on many factors such as the age and general condition of the subject, the particular agent or agents, and the like. Thus, it is not always possible to specify a quantified "effective amount." However, an appropriate "effective amount" in any subject case may be determined by one of ordinary skill in the art using routine experimentation. Also, as used herein, and unless specifically stated otherwise, an "effective amount" of an agent can also refer to an amount covering both therapeutically effective amounts and prophylactically effective amounts. An "effective amount" of an agent necessary to achieve a therapeutic effect may vary according to factors such as the age, sex, and weight of the subject. Dosage regimens can be adjusted to provide the optimum therapeutic response. For example, several divided doses may be administered daily, or the dose may be proportionally reduced as indicated by the exigencies of the therapeutic situation.

28. A "decrease" can refer to any change that results in a smaller amount of a symptom, disease, composition, condition, or activity. A substance is also understood to decrease the genetic output of a gene when the genetic output of the gene product with the substance is less relative to the output of the gene product without the substance. Also, for example, a decrease can be a change in the symptoms of a disorder such that the symptoms are less than previously observed. A decrease can be any individual, median, or average decrease in a condition, symptom, activity, composition in a statistically significant amount. Thus, the decrease can be a 1, 2, 3, 4, 5, 6, 7, 8, 9, 10, 15, 20, 25, 30, 35, 40, 45, 50, 55, 60, 65, 70, 75, 80, 85, 90, 95, or 100% decrease so long as the decrease is statistically significant.

29. "Inhibit," "inhibiting," and "inhibition" mean to decrease an activity, response, condition, disease, or other biological parameter. This can include but is not limited to the complete ablation of the activity, response, condition, or disease. This may also include, for example, a 10% reduction in the activity, response, condition, or disease as compared to the native or control level. Thus, the reduction can be a 10, 20, 30, 40, 50, 60, 70, 80, 90, 100%, or any amount of reduction in between as compared to native or control levels.

30. An "increase" can refer to any change that results in a greater amount of a symptom, disease, composition, condition or activity. An increase can be any individual, median, or average increase in a condition, symptom, activity, composition in a statistically significant amount. Thus, the increase can be a 1, 2, 3, 4, 5, 6, 7, 8, 9, 10, 15, 20, 25, 30, 35, 40, 45, 50, 55, 60, 65, 70, 75, 80, 85, 90, 95, or 100% increase so long as the increase is statistically significant.

31. "Pharmaceutically acceptable" component can refer to a component that is not biologically or otherwise undesirable, i.e., the component may be incorporated into a pharmaceutical formulation of the invention and administered to a subject as described herein without causing significant undesirable biological effects or interacting in a deleterious manner with any of the other components of the formulation in which it is contained. When used in reference to administration to a human, the term generally implies the component has met the required standards of toxicological and manufacturing testing or that it is included on the Inactive Ingredient Guide prepared by the U.S. Food and Drug Administration.

32. "Pharmaceutically acceptable carrier" (sometimes referred to as a "carrier") means a carrier or excipient that is useful in preparing a pharmaceutical or therapeutic composition that is generally safe and non-toxic and includes a carrier that is acceptable for veterinary and/or human pharmaceutical or therapeutic use. The terms "carrier" or "pharmaceutically acceptable carrier" can include, but are not limited to, phosphate buffered saline solution, water, emulsions (such as an oil/water or water/oil emulsion) and/or various types of wetting agents. As used herein, the term "carrier" encompasses, but is not limited to, any excipient, diluent, filler, salt, buffer, stabilizer, solubilizer, lipid, stabilizer, or other material well known in the art for use in pharmaceutical formulations and as described further herein.

33. "Pharmacologically active" (or simply "active"), as in a "pharmacologically active" derivative or analog, can refer to a derivative or analog (e.g., a salt, ester, amide, conjugate, metabolite, isomer, fragment, etc.) having the same type of pharmacological activity as the parent compound and approximately equivalent in degree.

34. "Polymer" refers to a relatively high molecular weight organic compound, natural or synthetic, whose structure can be represented by a repeated small unit, the monomer. Non-limiting examples of polymers include polyethylene, rubber, cellulose. Synthetic polymers are typically formed by addition or condensation polymerization of monomers. The term "copolymer" refers to a polymer formed from two or more different repeating units (monomer residues). By way of example and without limitation, a copolymer can be an alternating copolymer, a random copolymer, a block copolymer, or a graft copolymer. It is also contemplated that, in certain aspects, various block segments of a block copolymer can themselves comprise copolymers. The term "polymer" encompasses all forms of polymers including, but not limited to, natural polymers, synthetic polymers, homopolymers, heteropolymers or copolymers, addition polymers, etc.

35. "Therapeutic agent" refers to any composition that has a beneficial biological effect. Beneficial biological effects include both therapeutic effects, e.g., treatment of a disorder or other undesirable physiological condition, and prophylactic effects, e.g., prevention of a disorder or other undesirable physiological condition (e.g., a non-immunogenic cancer). The terms also encompass pharmaceutically acceptable, pharmacologically active derivatives of beneficial agents specifically mentioned herein, including, but not limited to, salts, esters, amides, proagents, active metabolites, isomers, fragments, analogs, and the like. When the terms "therapeutic agent" is used, then, or when a particular agent is specifically identified, it is to be understood that the term includes the agent per se as well as pharmaceutically acceptable, pharmacologically active salts, esters, amides, proagents, conjugates, active metabolites, isomers, fragments, analogs, etc.

36. "Therapeutically effective amount" or "therapeutically effective dose" of a composition (e.g. a composition comprising an agent) refers to an amount that is effective to achieve a desired therapeutic result. In some embodiments, a desired therapeutic result is the control of type I diabetes. In some embodiments, a desired therapeutic result is the control of obesity. Therapeutically effective amounts of a given therapeutic agent will typically vary with respect to factors such as the type and severity of the disorder or disease being treated and the age, gender, and weight of the subject. The term can also refer to an amount of a therapeutic agent, or a rate of delivery of a therapeutic agent (e.g., amount over time), effective to facilitate a desired therapeutic effect, such as pain relief. The precise desired therapeutic effect will vary according to the condition to be treated, the tolerance of the subject, the agent and/or agent formulation to be administered (e.g., the potency of the therapeutic agent, the concentration of agent in the formulation, and the like), and a variety of other factors that are appreciated by those of ordinary skill in the art. In some instances, a desired biological or medical response is achieved following administration of multiple dosages of the composition to the subject over a period of days, weeks, or years.

37. Throughout this application, various publications are referenced. The disclosures of these publications in their entireties are hereby incorporated by reference into this application in order to more fully describe the state of the art to which this pertains. The references disclosed are also individually and specifically incorporated by reference herein for the material contained in them that is discussed in the sentence in which the reference is relied upon.

## B. Compositions

38. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular adipocyte encapsulated anti-cancer drug is disclosed and discussed and a number of modifications that can be made to a number of molecules including the adipocyte encapsulated anti-cancer drug are discussed, specifically contemplated is each and every combination and permutation of adipocyte encapsulated anti-cancer drug and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

39. In this work, adipocytes were utilized as drug delivery depot for sustained release of chemotherapeutics to enhance anticancer efficacy and simultaneously regulate the tumor immune microenvironment to promote effector CD4 and CD8 T cell infiltration (Fig. 1a). Thus, in one aspect, disclosed herein are engineered adipocytes comprising an anti-cancer agent and a conjugated fatty acid.

40. It is understood and herein in contemplated that the disclosed engineered adipocytes comprise anti-cancer agents for the purpose of delivering sustained therapeutic release directly to the cancer cell. It is understood and herein contemplated that the therapeutic anti-cancer agent can comprise an antibody, small molecule, peptide, polypeptide, peptide mimetic, polymer, or nucleic acid. For example, the therapeutic agent cargo one or more chemotherapeutic agents. Chemotherapeutic agents that can be used in the disclosed hydrogel matrixes can comprise any anti-cancer agent known in the art, the including, but not limited to Abemaciclib, Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Afatinib Dimaleate, Afinitor (Everolimus), Akynzeo (Netupitant and Palonosetron Hydrochloride), Aldara (Imiquimod), Aldesleukin, Alecensa (Alectinib), Alectinib, Alemtuzumab, Alimta (Pemetrexed Disodium), Aliqopa (Copanlisib Hydrochloride), Alkeran for Injection (Melphalan Hydrochloride), Alkeran Tablets (Melphalan), Aloxi (Palonosetron Hydrochloride), Alunbrig (Brigatinib), Ambochlorin (Chlorambucil), Amboclorin Chlorambucil), Amifostine, Aminolevulinic Acid, Anastrozole, Aprepitant, Aredia (Pamidronate Disodium), Arimidex (Anastrozole), Aromasin (Exemestane),Arranon (Nelarabine), Arsenic Trioxide, Arzerra (Ofatumumab), Asparaginase Erwinia chrysanthemi, Atezolizumab, Avastin (Bevacizumab), Avelumab, Axitinib, Azacitidine, Bavencio (Avelumab), BEACOPP, Becenum (Carmustine), Beleodaq (Belinostat), Belinostat, Bendamustine Hydrochloride, BEP, Besponsa (Inotuzumab Ozogamicin) , Bevacizumab, Bexarotene, Bexxar (Tositumomab and Iodine I 131 Tositumomab), Bicalutamide, BiCNU (Carmustine), Bleomycin, Blinatumomab, Blincyto (Blinatumomab), Bortezomib, Bosulif (Bosutinib), Bosutinib, Brentuximab Vedotin, Brigatinib, BuMel, Busulfan, Busulfex (Busulfan), Cabazitaxel, Cabometyx (Cabozantinib-S-Malate), Cabozantinib-S-Malate, CAF, Campath (Alemtuzumab), Camptosar , (Irinotecan Hydrochloride), Capecitabine, CAPOX, Carac (Fluorouracil--Topical), Carboplatin, CARBOPLATIN-TAXOL, Carfilzomib, Carmubris (Carmustine), Carmustine, Carmustine Implant, Casodex (Bicalutamide), CEM, Ceritinib, Cerubidine (Daunorubicin Hydrochloride), Cervarix (Recombinant HPV Bivalent Vaccine), Cetuximab, CEV, Chlorambucil, CHLORAMBUCIL-PREDNISONE, CHOP, Cisplatin, Cladribine, Clafen (Cyclophosphamide), Clofarabine, Clofarex (Clofarabine), Clolar (Clofarabine), CMF, Cobimetinib, Cometriq (Cabozantinib-S-Malate), Copanlisib Hydrochloride, COPDAC, COPP, COPP-ABV, Cosmegen (Dactinomycin), Cotellic (Cobimetinib), Crizotinib, CVP, Cyclophosphamide, Cyfos (Ifosfamide), Cyramza (Ramucirumab), Cytarabine, Cytarabine Liposome, Cytosar-U (Cytarabine), Cytoxan (Cyclophosphamide), Dabrafenib, Dacarbazine, Dacogen (Decitabine), Dactinomycin, Daratumumab, Darzalex (Daratumumab), Dasatinib, Daunorubicin Hydrochloride, Daunorubicin Hydrochloride and Cytarabine Liposome, Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Durvalumab, Efudex (Fluorouracil--Topical), Elitek (Rasburicase), Ellence (Epirubicin Hydrochloride), Elotuzumab, Eloxatin (Oxaliplatin), Eltrombopag Olamine, Emend (Aprepitant), Empliciti (Elotuzumab), Enasidenib Mesylate, Enzalutamide, Epirubicin Hydrochloride , EPOCH, Erbitux (Cetuximab), Eribulin Mesylate, Erivedge (Vismodegib), Erlotinib Hydrochloride, Erwinaze (Asparaginase Erwinia chrysanthemi) , Ethyol (Amifostine), Etopophos (Etoposide Phosphate), Etoposide, Etoposide Phosphate, Evacet (Doxorubicin Hydrochloride Liposome), Everolimus, Evista , (Raloxifene Hydrochloride), Evomela (Melphalan Hydrochloride), Exemestane, 5-FU (Fluorouracil Injection), 5-FU (Fluorouracil-Topical), Fareston (Toremifene), Farydak (Panobinostat), Faslodex (Fulvestrant), FEC, Femara (Letrozole), Filgrastim, Fludara (Fludarabine Phosphate), Fludarabine Phosphate, Fluoroplex (Fluorouracil--Topical), Fluorouracil Injection, Fluorouracil--Topical, Flutamide, Folex (Methotrexate), Folex PFS (Methotrexate), FOLFIRI, FOLFIRI-BEVACIZUMAB,          FOLFIRI-CETUXIMAB, FOLFIRINOX, FOLFOX, Folotyn (Pralatrexate), FU-LV, Fulvestrant, Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, GEMCITABINE-CISPLATIN, GEMCITABINE-OXALIPLATIN, Gemtuzumab Ozogamicin, Gemzar (Gemcitabine Hydrochloride), Gilotrif (Afatinib Dimaleate), Gleevec (Imatinib Mesylate), Gliadel (Carmustine Implant), Gliadel wafer (Carmustine Implant), Glucarpidase, Goserelin Acetate, Halaven (Eribulin Mesylate), Hemangeol (Propranolol Hydrochloride), Herceptin (Trastuzumab), HPV Bivalent Vaccine, Recombinant, HPV Nonavalent Vaccine, Recombinant, HPV Quadrivalent Vaccine, Recombinant, Hycamtin (Topotecan Hydrochloride), Hydrea (Hydroxyurea), Hydroxyurea, Hyper-CVAD, Ibrance (Palbociclib), Ibritumomab Tiuxetan, Ibrutinib, ICE, Iclusig (Ponatinib Hydrochloride), Idamycin (Idarubicin Hydrochloride), Idarubicin Hydrochloride, Idelalisib, Idhifa (Enasidenib Mesylate), Ifex (Ifosfamide), Ifosfamide, Ifosfamidum (Ifosfamide), IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Recombinant, Interleukin-2 (Aldesleukin), Intron A (Recombinant Interferon Alfa-2b), Iodine I 131 Tositumomab and Tositumomab, Ipilimumab, Iressa (Gefitinib), Irinotecan Hydrochloride, Irinotecan Hydrochloride Liposome, Istodax (Romidepsin), Ixabepilone, Ixazomib Citrate, Ixempra (Ixabepilone), Jakafi (Ruxolitinib Phosphate), JEB, Jevtana (Cabazitaxel), Kadcyla (Ado-Trastuzumab Emtansine), Keoxifene (Raloxifene Hydrochloride), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Leustatin (Cladribine), Levulan (Aminolevulinic Acid), Linfolizin (Chlorambucil), LipoDox (Doxorubicin Hydrochloride Liposome), Lomustine, Lonsurf (Trifluridine and Tipiracil Hydrochloride), Lupron (Leuprolide Acetate), Lupron Depot (Leuprolide Acetate), Lupron Depot-Ped (Leuprolide Acetate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Melphalan, Melphalan Hydrochloride, Mercaptopurine, Mesna, Mesnex (Mesna), Methazolastone (Temozolomide), Methotrexate, Methotrexate LPF (Methotrexate), Methylnaltrexone Bromide, Mexate (Methotrexate), Mexate-AQ (Methotrexate), Midostaurin, Mitomycin C, Mitoxantrone Hydrochloride, Mitozytrex (Mitomycin C), MOPP, Mozobil (Plerixafor), Mustargen (Mechlorethamine Hydrochloride) , Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neosar (Cyclophosphamide), Neratinib Maleate, Nerlynx (Neratinib Maleate), Netupitant and Palonosetron Hydrochloride, Neulasta (Pegfilgrastim), Neupogen (Filgrastim), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nolvadex (Tamoxifen Citrate), Nplate (Romiplostim), Obinutuzumab, Odomzo (Sonidegib), OEPA, Ofatumumab, OFF, Olaparib, Olaratumab, Omacetaxine Mepesuccinate, Oncaspar (Pegaspargase), Ondansetron Hydrochloride, Onivyde (Irinotecan Hydrochloride Liposome), Ontak (Denileukin Diftitox), Opdivo (Nivolumab), OPPA, Osimertinib, Oxaliplatin, Paclitaxel, Paclitaxel Albumin-stabilized Nanoparticle Formulation, PAD, Palbociclib, Palifermin, Palonosetron Hydrochloride, Palonosetron Hydrochloride and Netupitant, Pamidronate Disodium, Panitumumab, Panobinostat, Paraplat (Carboplatin), Paraplatin (Carboplatin), Pazopanib Hydrochloride, PCV, PEB, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Portrazza (Necitumumab), Pralatrexate, Prednisone, Procarbazine Hydrochloride , Proleukin (Aldesleukin), Prolia (Denosumab), Promacta (Eltrombopag Olamine), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, R-CHOP, R-CVP, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), R-EPOCH, Revlimid (Lenalidomide), Rheumatrex (Methotrexate), Ribociclib, R-ICE, Rituxan (Rituximab), Rituxan Hycela (Rituximab and Hyaluronidase Human), Rituximab, Rituximab and , Hyaluronidase Human, ,Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), STANFORD V, Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), TAC, Tafinlar (Dabrafenib), Tagrisso (Osimertinib), Talc, Talimogene Laherparepvec, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Tecentriq , (Atezolizumab), Temodar (Temozolomide), Temozolomide, Temsirolimus, Thalidomide, Thalomid (Thalidomide), Thioguanine, Thiotepa, Tisagenlecleucel, Tolak (Fluorouracil--Topical), Topotecan Hydrochloride, Toremifene, Torisel (Temsirolimus), Tositumomab and Iodine I 131 Tositumomab, Totect (Dexrazoxane Hydrochloride), TPF, Trabectedin, Trametinib, Trastuzumab, Treanda (Bendamustine Hydrochloride), Trifluridine and Tipiracil Hydrochloride, Trisenox (Arsenic Trioxide), Tykerb (Lapatinib Ditosylate), Unituxin (Dinutuximab), Uridine Triacetate, VAC, Vandetanib, VAMP, Varubi (Rolapitant Hydrochloride), Vectibix (Panitumumab), VeIP, Velban (Vinblastine Sulfate), Velcade (Bortezomib), Velsar (Vinblastine Sulfate), Vemurafenib, Venclexta (Venetoclax), Venetoclax, Verzenio (Abemaciclib), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, VIP, Vismodegib, Vistogard (Uridine Triacetate), Voraxaze (Glucarpidase), Vorinostat, Votrient (Pazopanib Hydrochloride), Vyxeos (Daunorubicin Hydrochloride and Cytarabine Liposome), Wellcovorin (Leucovorin Calcium), Xalkori (Crizotinib), Xeloda (Capecitabine), XELIRI, XELOX, Xgeva (Denosumab), Xofigo (Radium 223 Dichloride), Xtandi (Enzalutamide), Yervoy (Ipilimumab), Yondelis (Trabectedin), Zaltrap (Ziv-Aflibercept), Zarxio (Filgrastim), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zofran (Ondansetron Hydrochloride), Zoladex (Goserelin Acetate), Zoledronic Acid, Zolinza (Vorinostat), Zometa (Zoledronic Acid), Zydelig (Idelalisib), Zykadia (Ceritinib), and/or Zytiga (Abiraterone Acetate), a PD-1 inhibitor, a PD-L1 inhibitor, or CTLA-4 inhibitor (such as, for example, nivolumab, pembrolizumab, pidilizumab, BMS-936559, Atezolizumab, Durvalumab, or Avelumab), or any salts, esters, amides, prodrugs, proagents, conjugates, active metabolites, isomers, fragments, and/or analogs thereof. It is understood and herein contemplated that some fatty acids (such as, for example, docosahexaenoic acid (DHA) and eicosapentaenoic acid (EPA)) have anti-cancer therapeutic effects and can be used in the disclosed methods and compositions as an anti-cancer agent along side the conjugated fatty acid of the engineered adipocytes. Such anti-cancer fatty acids may also be employed in addition to any other anti-cancer agent disclosed herein.

41. It is further understood and herein contemplated that the engineered adipocytes can comprise more than one type of anti-cancer agent, blockade inhibitor, or immunomodulatory agent. For example, the nanoparticle can comprise any combination of 1, 2, 3, 4, 5, 6, 7, 8, 910, 11, 12, 13, 14, 15, 16, 17, 18, 19, or 20 anti-cancer agents, blockade inhibitors, or immunomodulatory agents.

42. The engineered adipocytes disclosed herein can be loaded with fatty acids to which the anti-cancer agent is conjugated. Conjugated fatty acids are polyunsaturated fatty acids comprising at least one double bond pair separated by only one single bond. In one aspect, the conjugated fatty acid can comprise one or more isomers of conjugated linoleic acid including, but not limited to 9cis, 11trans, 10trans, and/or 12cis. For example, in one aspect, the conjugated fatty acid comprises rumenic acid (9cis, 11 trans linoleic acid). Additional fatty acids for use in the disclosed methods and in the engineered adipocytes include, docosahexaenoic acid (DHA) and eicosapentaenoic acid (EPA).

43. In one aspect, the prodrug can be conjugated to the conjugated fatty acid via an environmentally reactive linker. A "linker" as used herein refers to a molecule that joins adjacent molecules. Generally a linker has no specific biological activity other than to join the adjacent molecules or to preserve some minimum distance or other spatial relationship between them. In some cases, the linker can be selected to influence or stabilize some property of the adjacent molecules, such as the folding, net charge, or hydrophobicity of the molecule. Examples of environmentally responsive linkers include, but are not limited to pH responsive linkers (for example, ester linkers, hydrazine, carboxy dimethylmaleic andhydride, orthoester, imine, β-thioproprionate, vinylether, and phophroamidate), enzymatic responsive linkers, glucose responsive linkers (such as, for example, boronic acid, ethylene glycol dimethacrylate, methylene bisacrylamide, Poly(ethylene glycol) diacrylate, and ethylene glycol dimethacrylate), or $H_2O_2$ or other reactive oxygen species responsive linkers (thioether, selenide, telluride, diselenide,, thioketal arylboronic ester, aminoacrylate, peroxalate ester, mesoporus silicon, and oligoproline). Linkers can also be peptide linkers in addition to any linker disclosed above.

44. It is understood and herein contemplated that lipid transport proteins provide fatty acids to cancer cells in the tumor microenvironment. By providing a lipid transport protein in the engineered adipocyte, the prodrug conjugated to a fatty acid can similarly be delivered to the cancer cell. Thus, in one aspect, disclosed herein are adipocytes of any preceding aspect further comprising a lipid transport protein. The lipid transport protein can be any lipid transport protein known in the art, including, but not limited to fatty-acid binding protein (FABP) 4 (FABP4), FABP1, FABP2, FABP3, FABP5, FABP6, FABP7. FABP8, FABP9, FABP11, FABP12, FABP 5-like 1, FABP 5-like 2, FABP 5-like 3, FABP 5-like 4, FABP 5-like 5, FABP 5-like 6, FABP 5-like 7, fatty acid transport protein (FATP) 1 (FATP1), FATP2, FATP3, FATP4, FATP5, and/or FATP6.

45. Also disclosed herein are methods of providing sustained release of an anti-cancer agent to a tumor comprising conjugating the anti-cancer agent to a conjugated fatty acid, encapsulating the conjugated anti-cancer agent in an adipocyte to make an engineered adipocyte, and delivering the engineered adipocyte to a tumor.

**1. Pharmaceutical carriers/Delivery of pharmaceutical products**

46. As described above, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

47. The compositions may be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

48. Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795

49. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo*. The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

### a) Pharmaceutically Acceptable Carriers

50. The compositions, including antibodies, can be used therapeutically in combination with a pharmaceutically acceptable carrier.

51. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

52. Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

53. Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

54. The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

55. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

56. Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

57. Compositions for oral administration include powders or granules, suspensions or solutions in water or non-aqueous media, capsules, sachets, or tablets. Thickeners, flavorings, diluents, emulsifiers, dispersing aids or binders may be desirable..

58. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### b) Therapeutic Uses

59. Effective dosages and schedules for administering the compositions may be determined empirically, and making such determinations is within the skill in the art. The dosage ranges for the administration of the compositions are those large enough to produce the desired effect in which the symptoms of the disorder are effected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any counterindications. Dosage can vary, and can be administered in one or more dose administrations daily, for one or several days. Guidance can be found in the literature for appropriate dosages for given classes of pharmaceutical products. For example, guidance in selecting appropriate doses for antibodies can be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 μg/kg to up to 100 mg/kg of body weight or more per day, depending on the factors mentioned above.

**C. Method of treating cancer**

60. The disclosed compositions can be used to treat any disease where uncontrolled cellular proliferation occurs such as cancers. Accordingly, in one aspect, disclosed herein are methods of treating, preventing, inhibiting, or reducing a cancer or metastasis comprising in a subject comprising administering to the subject one or more of the engineered adipocytes disclosed herein. For example, disclosed herein are methods of treating, preventing, inhibiting, or reducing a cancer or metastasis comprising in a subject comprising administering to the subject one or more engineered adipocytes comprising an anti-cancer prodrug (such as, for example, doxorubicin prodrug) and a conjugated fatty acid (such as, for example, one or more isomers of conjugated linoleic acid including, but not limited to 9cis, 11trans, 10trans, and/or 12cis).

61. A non-limiting list of different types of cancers that can be treated, inhibited, reduced and/or prevented using the disclosed engineered adipocytes is as follows: lymphomas (Hodgkins and non-Hodgkins), leukemias, carcinomas, carcinomas of solid tissues, squamous cell carcinomas, adenocarcinomas, sarcomas, gliomas, high grade gliomas, blastomas, neuroblastomas, plasmacytomas, histiocytomas, melanomas, adenomas, hypoxic tumours, myelomas, AIDS-related lymphomas or sarcomas, metastatic cancers, or cancers in general.

62. A representative but non-limiting list of cancers that the disclosed compositions can be used to treat is the following: lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, lung cancers such as small cell lung cancer and non-small cell lung cancer, neuroblastoma/glioblastoma, ovarian cancer, skin cancer, liver cancer, melanoma, squamous cell carcinomas of the mouth, throat, larynx, and lung, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers; testicular cancer; colon cancer, rectal cancer, prostatic cancer, or pancreatic cancer.
63. Chemotherapeutic agents that can be conjugated to a fatty acid encapsulated in the disclosed engineered adipocytes for treatment of a cancer in any of the methods disclosed herein can comprise any anti-cancer agent known in the art, the including, but not limited to Abemaciclib, Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Afatinib Dimaleate, Afinitor (Everolimus), Akynzeo (Netupitant and Palonosetron Hydrochloride), Aldara (Imiquimod), Aldesleukin, Alecensa (Alectinib), Alectinib, Alemtuzumab, Alimta (Pemetrexed Disodium), Aliqopa (Copanlisib Hydrochloride), Alkeran for Injection (Melphalan Hydrochloride), Alkeran Tablets (Melphalan), Aloxi (Palonosetron Hydrochloride), Alunbrig (Brigatinib), Ambochlorin (Chlorambucil), Amboclorin Chlorambucil), Amifostine, Aminolevulinic Acid, Anastrozole, Aprepitant, Aredia (Pamidronate Disodium), Arimidex (Anastrozole), Aromasin (Exemestane),Arranon (Nelarabine), Arsenic Trioxide, Arzerra (Ofatumumab), Asparaginase Erwinia chrysanthemi, Atezolizumab, Avastin (Bevacizumab), Avelumab, Axitinib, Azacitidine, Bavencio (Avelumab), BEACOPP, Becenum (Carmustine), Beleodaq (Belinostat), Belinostat, Bendamustine Hydrochloride, BEP, Besponsa (Inotuzumab Ozogamicin) , Bevacizumab, Bexarotene, Bexxar (Tositumomab and Iodine I 131 Tositumomab), Bicalutamide, BiCNU (Carmustine), Bleomycin, Blinatumomab, Blincyto (Blinatumomab), Bortezomib, Bosulif (Bosutinib), Bosutinib, Brentuximab Vedotin, Brigatinib, BuMel, Busulfan, Busulfex (Busulfan), Cabazitaxel, Cabometyx (Cabozantinib-S-Malate), Cabozantinib-S-Malate, CAF, Campath (Alemtuzumab), Camptosar , (Irinotecan Hydrochloride), Capecitabine, CAPOX, Carac (Fluorouracil--Topical), Carboplatin, CARBOPLATIN-TAXOL, Carfilzomib, Carmubris (Carmustine), Carmustine, Carmustine Implant, Casodex (Bicalutamide), CEM, Ceritinib, Cerubidine (Daunorubicin Hydrochloride), Cervarix (Recombinant HPV Bivalent Vaccine), Cetuximab, CEV, Chlorambucil, CHLORAMBUCIL-PREDNISONE, CHOP, Cisplatin, Cladribine, Clafen (Cyclophosphamide), Clofarabine, Clofarex (Clofarabine), Clolar (Clofarabine), CMF, Cobimetinib, Cometriq (Cabozantinib-S-Malate), Copanlisib Hydrochloride, COPDAC, COPP, COPP-ABV, Cosmegen (Dactinomycin), Cotellic (Cobimetinib), Crizotinib, CVP, Cyclophosphamide, Cyfos (Ifosfamide), Cyramza (Ramucirumab), Cytarabine, Cytarabine Liposome, Cytosar-U (Cytarabine), Cytoxan (Cyclophosphamide), Dabrafenib, Dacarbazine, Dacogen (Decitabine), Dactinomycin, Daratumumab, Darzalex (Daratumumab), Dasatinib, Daunorubicin Hydrochloride, Daunorubicin Hydrochloride and Cytarabine Liposome, Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Durvalumab, Efudex (Fluorouracil--Topical), Elitek (Rasburicase), Ellence (Epirubicin Hydrochloride), Elotuzumab, Eloxatin (Oxaliplatin), Eltrombopag Olamine, Emend (Aprepitant), Empliciti (Elotuzumab), Enasidenib Mesylate, Enzalutamide, Epirubicin Hydrochloride , EPOCH, Erbitux (Cetuximab), Eribulin Mesylate, Erivedge (Vismodegib), Erlotinib Hydrochloride, Erwinaze (Asparaginase Erwinia chrysanthemi) , Ethyol (Amifostine), Etopophos (Etoposide Phosphate), Etoposide, Etoposide Phosphate, Evacet (Doxorubicin Hydrochloride Liposome), Everolimus, Evista , (Raloxifene Hydrochloride), Evomela (Melphalan Hydrochloride), Exemestane, 5-FU (Fluorouracil Injection), 5-FU (Fluorouracil-Topical), Fareston (Toremifene), Farydak (Panobinostat), Faslodex (Fulvestrant), FEC, Femara (Letrozole), Filgrastim, Fludara (Fludarabine Phosphate), Fludarabine Phosphate, Fluoroplex (Fluorouracil--Topical), Fluorouracil Injection, Fluorouracil--Topical, Flutamide, Folex (Methotrexate), Folex PFS (Methotrexate), FOLFIRI, FOLFIRI-BEVACIZUMAB, FOLFIRI-CETUXIMAB, FOLFIRINOX, FOLFOX, Folotyn (Pralatrexate), FU-LV, Fulvestrant, Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, GEMCITABINE-CISPLATIN, GEMCITABINE-OXALIPLATIN, Gemtuzumab Ozogamicin, Gemzar (Gemcitabine Hydrochloride), Gilotrif (Afatinib Dimaleate), Gleevec (Imatinib Mesylate), Gliadel (Carmustine Implant), Gliadel wafer (Carmustine Implant), Glucarpidase, Goserelin Acetate, Halaven (Eribulin Mesylate), Hemangeol (Propranolol Hydrochloride), Herceptin (Trastuzumab), HPV Bivalent Vaccine, Recombinant, HPV Nonavalent Vaccine, Recombinant, HPV Quadrivalent Vaccine, Recombinant, Hycamtin (Topotecan Hydrochloride), Hydrea (Hydroxyurea), Hydroxyurea, Hyper-CVAD, Ibrance (Palbociclib), Ibritumomab Tiuxetan, Ibrutinib, ICE, Iclusig (Ponatinib Hydrochloride), Idamycin (Idarubicin Hydrochloride), Idarubicin Hydrochloride, Idelalisib, Idhifa (Enasidenib Mesylate), Ifex (Ifosfamide), Ifosfamide, Ifosfamidum (Ifosfamide), IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Recombinant, Interleukin-2 (Aldesleukin), Intron A (Recombinant Interferon Alfa-2b), Iodine I 131 Tositumomab and Tositumomab, Ipilimumab, Iressa (Gefitinib), Irinotecan Hydrochloride, Irinotecan Hydrochloride Liposome, Istodax (Romidepsin), Ixabepilone, Ixazomib Citrate, Ixempra (Ixabepilone), Jakafi (Ruxolitinib Phosphate), JEB, Jevtana (Cabazitaxel), Kadcyla (Ado-Trastuzumab Emtansine), Keoxifene (Raloxifene Hydrochloride), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Leustatin (Cladribine), Levulan (Aminolevulinic Acid), Linfolizin (Chlorambucil), LipoDox (Doxorubicin Hydrochloride Liposome), Lomustine, Lonsurf (Trifluridine and Tipiracil Hydrochloride), Lupron (Leuprolide Acetate), Lupron Depot (Leuprolide Acetate), Lupron Depot-Ped (Leuprolide Acetate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Melphalan, Melphalan Hydrochloride, Mercaptopurine, Mesna, Mesnex (Mesna), Methazolastone (Temozolomide), Methotrexate, Methotrexate LPF (Methotrexate), Methylnaltrexone Bromide, Mexate (Methotrexate), Mexate-AQ (Methotrexate), Midostaurin, Mitomycin C, Mitoxantrone Hydrochloride, Mitozytrex (Mitomycin C), MOPP, Mozobil (Plerixafor), Mustargen (Mechlorethamine Hydrochloride), Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neosar (Cyclophosphamide), Neratinib Maleate, Nerlynx (Neratinib Maleate), Netupitant and Palonosetron Hydrochloride, Neulasta (Pegfilgrastim), Neupogen (Filgrastim), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nolvadex (Tamoxifen Citrate), Nplate (Romiplostim), Obinutuzumab, Odomzo (Sonidegib), OEPA, Ofatumumab, OFF, Olaparib, Olaratumab, Omacetaxine Mepesuccinate, Oncaspar (Pegaspargase), Ondansetron Hydrochloride, Onivyde (Irinotecan Hydrochloride Liposome), Ontak (Denileukin Diftitox), Opdivo (Nivolumab), OPPA, Osimertinib, Oxaliplatin, Paclitaxel, Paclitaxel Albumin-stabilized Nanoparticle Formulation, PAD, Palbociclib, Palifermin, Palonosetron Hydrochloride, Palonosetron Hydrochloride and Netupitant, Pamidronate Disodium, Panitumumab, Panobinostat, Paraplat (Carboplatin), Paraplatin (Carboplatin), Pazopanib Hydrochloride, PCV, PEB, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Portrazza (Necitumumab), Pralatrexate, Prednisone, Procarbazine Hydrochloride , Proleukin (Aldesleukin), Prolia (Denosumab), Promacta (Eltrombopag Olamine), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, R-CHOP, R-CVP, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), R-EPOCH, Revlimid (Lenalidomide), Rheumatrex (Methotrexate), Ribociclib, R-ICE, Rituxan (Rituximab), Rituxan Hycela (Rituximab and Hyaluronidase Human), Rituximab, Rituximab and , Hyaluronidase Human, ,Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), STANFORD V, Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), TAC, Tafinlar (Dabrafenib), Tagrisso (Osimertinib), Talc, Talimogene Laherparepvec, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Tecentriq , (Atezolizumab), Temodar (Temozolomide), Temozolomide, Temsirolimus, Thalidomide, Thalomid (Thalidomide), Thioguanine, Thiotepa, Tisagenlecleucel, Tolak (Fluorouracil--Topical), Topotecan Hydrochloride, Toremifene, Torisel (Temsirolimus), Tositumomab and Iodine I 131 Tositumomab, Totect (Dexrazoxane Hydrochloride), TPF, Trabectedin, Trametinib, Trastuzumab, Treanda (Bendamustine Hydrochloride), Trifluridine and Tipiracil Hydrochloride, Trisenox (Arsenic Trioxide), Tykerb (Lapatinib Ditosylate), Unituxin (Dinutuximab), Uridine Triacetate, VAC, Vandetanib, VAMP, Varubi (Rolapitant Hydrochloride), Vectibix (Panitumumab), VeIP, Velban (Vinblastine Sulfate), Velcade (Bortezomib), Velsar (Vinblastine Sulfate), Vemurafenib, Venclexta (Venetoclax), Venetoclax, Verzenio (Abemaciclib), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, VIP, Vismodegib, Vistogard (Uridine Triacetate), Voraxaze (Glucarpidase), Vorinostat, Votrient (Pazopanib Hydrochloride), Vyxeos (Daunorubicin Hydrochloride and Cytarabine Liposome), Wellcovorin (Leucovorin Calcium), Xalkori (Crizotinib), Xeloda (Capecitabine), XELIRI, XELOX, Xgeva (Denosumab), Xofigo (Radium 223 Dichloride), Xtandi (Enzalutamide), Yervoy (Ipilimumab), Yondelis (Trabectedin), Zaltrap (Ziv-Aflibercept), Zarxio (Filgrastim), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zofran (Ondansetron Hydrochloride), Zoladex (Goserelin Acetate), Zoledronic Acid, Zolinza (Vorinostat), Zometa (Zoledronic Acid), Zydelig (Idelalisib), Zykadia (Ceritinib), and/or Zytiga (Abiraterone Acetate), a PD-1 inhibitor, a PD-L1 inhibitor, or CTLA-4 inhibitor (such as, for example, nivolumab, pembrolizumab, pidilizumab, BMS-936559, Atezolizumab, Durvalumab, or Avelumab), or any salts, esters, amides, prodrugs, proagents, conjugates, active metabolites, isomers, fragments, and/or analogs thereof. Thus, in one aspect, are methods of treating, preventing, inhibiting, and/or reducing a cancer or metastasis in a subject comprising administering to the subject one or more engineered adipocytes comprising doxorubicin and a conjugated fatty acid (such as, for example, one or more isomers of conjugated linoleic acid including, but not limited to 9cis, 11trans, 10trans, and/or 12cis). In one aspect, disclosed herein are engineered adipocytes for use in methods of treating, preventing, inhibiting, and/or reducing a cancer or metastasis in a subject comprising administering to the subject one or more engineered adipocytes wherein the anti-cancer prodrug comprises doxorubicin prodrug and the conjugated linoleic acid comprises rumenic acid (9cis, 11 trans linoleic acid).

64. It is understood and herein contemplated that the fatty acid conjugated anti-cancer agent that is encapsulated by the engineered adipocyte can be designed to be bioresponsive to the microenvironment of the tumor and release the anti-cancer agent, blockade inhibitor, or immunomodulatory agent upon exposure to factors within the microenvironment such as, for example reactive oxygen species or pH. In one aspect, it is contemplated herein that the bioresponsive engineered adipocyte can be designed to release the anti-cancer agent, blockade inhibitor, or immunomodulatory agent into the tumor microenvironment for at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42,4 3,44, 45, 46, 47, 48, 49, 50, 51, 52, 53, 54, 55, 56, 57, 58, 59, 60, 65, 70, 75, 80, 85, or 90 days.

65. "Treat," "treating," "treatment," and grammatical variations thereof as used herein, include the administration of a composition with the intent or purpose of partially or completely preventing, delaying, curing, healing, alleviating, relieving, altering, remedying, ameliorating, improving, stabilizing, mitigating, and/or reducing the intensity or frequency of one or more a diseases or conditions, a symptom of a disease or condition, or an underlying cause of a disease or condition. Treatments according to the invention may be applied preventively, prophylactically, pallatively or remedially. Prophylactic treatments are administered to a subject prior to onset (e.g., before obvious signs of cancer), during early onset (e.g., upon initial signs and symptoms of cancer), or after an established development of cancer. Prophylactic administration can occur for day(s) to years prior to the manifestation of symptoms of an infection.

66. In one aspect, the disclosed methods of treating, preventing, inhibiting, or reducing a cancer or metastasis comprising administering to a subject any of engineered adipocytes or pharmaceutical compositions comprising said engineered adipocytes disclosed herein can comprise administration of the engineered adipocyte or pharmaceutical compositions at any frequency appropriate for the treatment of the particular cancer in the subject. For example, engineered adipocytes or pharmaceutical compositions can be administered to the patient at least once every 12, 14, 16, 18, 20, 22, 24, 26, 28, 30, 32, 34, 36, 38, 40, 42, 44, 46, 48 hours, once every 3, 4, 5, 6, 7, 8, 9, 10, 11, 12, 13, 14, 15, 16, 17, 18, 19, 20, 21, 22, 23, 24, 25, 26, 27, 28, 29, 30, 31 days, once every 2, 3, 4, 5, 6, 7, 8, 9, 10, 11, or 12 months. In one aspect, the therapeutic agent delivery vehicles or pharmaceutical compositions are administered at least 1, 2, 3, 4, 5, 6, 7 times per week.

## D. Examples

67. The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1

#### a) Results

68. In this work, adipocytes were utilized as drug delivery depot for sustained release of chemotherapeutics to enhance anticancer efficacy and simultaneously regulate the tumor immune microenvironment to promote effector CD4 and CD8 T cell infiltration (Fig. 1a). A pH and ROS responsive doxorubicin prodrug (pDox) was synthesized (Fig. 1b) and encapsulated into adipocytes with rumenic acid (RA), which enhanced the compatibility of Dox to adipocytes and further facilitated the transport of Dox to tumor cells through the lipid metabolism pathway. These engineered adipocytes can deliver pDox and RA into cancer cells with effects for cell killing and immune regulation (Fig. 1c). In the tumor microenvironment, several tumor promoting adipokines were downregulated by these engineered adipocytes and tumor inhibition immunity was established (Fig. 1d).

69. 3T3-L1 cell-differentiated adipocytes were co-cultured with several cancer cell lines in a transwell system. As expected, normal adipocytes promoted these cells growth (Fig. 1e-h). Meanwhile, adipokine profiling showed highly overexpressed VEGF and resistin expression in co-cultured medium and lipocalin-2 in adipocytes that facilitated cell growth and metastasis. In order to reverse the malignant role of adipocytes in tumor progression, we encapsulated RA, also named 9Z, 11E-conjugated linoleic acid, as an anti-cancer fatty acid to be encapsulated into adipocytes during their differentiation. As one of the most abundant isomers of conjugated linoleic acid (CLA), RA has been demonstrated to suppress breast, liver, and prostate cancer cell growth. According to the results, similar cytotoxicity of RA, CLA and another CLA isomer 10E, 12Z-CLA was observed with no obvious synergistic effect between RA and 10E, 12Z-CLA. B16F10 cell growth was inhibited when co-cultured with RA (RA@adipocyte) or CLA loaded adipocytes (CLA@adipocyte) in a transwell system (Fig. 1i). However, as a mixture of several isomers, CLA@adipocytes could not suppress E0771 cell growth (Fig. 1j), indicating different functions of different CLA isomers.

70. Several tumor promoting adipokines are secreted by TAA for inhibiting anti-cancer immune cell recruitment and guiding tumor metastasis. The data showed that different fatty acid encapsulated in adipocytes can affect adipokines secretion and their role on cancer cell growth. With significantly decreased resistin secretion, it can be the most important target of CLA@adipocyte and RA@adipocyte for suppressing tumor cell growth and metastasis. Interestingly, even though VEGF was highly overexpressed in RA@adipocyte medium, it dropped to normal concentration when co-cultured with B16F10 cells. Furthermore, expression of lipocalin-2 in adipocytes was significantly decreased in RA@adipocytes, while MCP-1 was overexpressed, which contributes to the recruitment of monocytes at the early stage of melanoma. However, there was no significant difference of MCP-1 concentration among co-cultured medium of B16F10 with CLA@adipocyte, RA@adipocyte, or normal adipocyte.

71. Recently, immune checkpoint blockage for programmed death-ligand 1 (PD-L1) showed promising clinical outcomes. A switch from white fat to brown fat, a phenomenon termed white adipose tissue browning, caused by TAA dysfunction promoted the expression of PD-L1 on brown adipocytes. However, the influence of adipocytes on cancer cell PD-L1 expression remains largely unknown. Other than testing the function of RA@adipocyte on adipokine secretion, its role on regulating immune cells was elucidated. In a transwell system, RA@adipocytes and CLA@adipocyte can suppress PD-L1 expression of B16F10 cells (Fig. 1k), thus facilitating the infiltration and activation of T-lymphocytes. This effect was partially reversed by BMS309403, an FABP4 inhibitor (iFABP4), indicating the crucial role of RA and CLA during the crosstalk between adipocyte and cancer cells. The results also indicated that 10E, 12Z-CLA loaded adipocytes or a mixture of RA and 10E, 12Z-CLA loaded adipocytes could not downregulate PD-L1 expression as effectively as RA@adipocyte. Next, the antitumour effects of RA@adipocytes were evaluated using B16F10 mouse melanoma tumor model (Fig. 3a). RA@adipocytes delayed tumor growth significantly in terms of the tumor size and survival rate (Fig. 2a, 2b, 2c, and 3c), while the body weight was not affected by this intratumoural injection of RA@adipocytes (Fig. 3b). Two days after the second injection of RA@adipocytes, tumours were harvested and analyzed by flow cytometry. PD-L1 was significantly downregulated in tumor cells from the mice received RA@adipocytes (Fig. 2d and 3d). As a result, marked infiltration of CD8[+] T cells in tumor was detected in RA@adipocytes treated group compared with control (Fig. 2e and 3e). Meanwhile, RA@adipocyte treated group showed a remarkble decrease of regulatory T cell (Treg) population (Fig. 2f and 3f). These data demonstrated that RA@adipocytes was able to suppress tumor growth and promote an immunogenic tumor phenotype.

72. The potency of RA@adipocytes was also investigated in a tumor resection model. Followed by encapsulation into a fibrin gel, these adipocytes were directly injected into the resection cavity. RA@adipocytes was able to delay tumor recurrence and growth (Fig. 2g 2h, 2i, 4a, and 4b) with no effects on the body weight (Fig. 4c). One week after RA@adipocytes administration, the population of PD-L1 positive cells, CD8[+] T cells, and Tregs were analyzed by flow cytometry. Significantly lower PD-L1 (Fig. 2j and 4d) expression was detected compared to control groups, thereby enhanced the population of CD8[+] T cells (Fig.2k and 4e) and decreased Tregs (Fig. 2l, and 4f) in tumor.

73. Although remaining one of the mainstays in cancer treatment, the application of chemotherapy is limited by severe side effects. To enhance the therapeutic selectivity, stimuli-responsive prodrugs or drug delivery systems have been verified as promising strategies. Some studies indicated at least 10-fold higher ROS concentration in tumor compared with normal cells. To further improve the therapeutic outcome of RA@adipocytes with diminished side effects, a doxorubicin (Dox) prodrug was synthesized by conjugating doxorubicin to oleic acid with a benzene boronic acid-based ROS responsive linker (Fig. 5). Upon oxidation of 10 mM $H_2O_2$, pDox was converted to Dox within 48 h. UV and fluorescent spectrums of pDox were further characterized compared with Dox. It was understood that lipid conjugation in pDox can enhance the uptake of cancer cells through the lipid metabolism pathway depending on FABP4, which was a key promoter for breast and ovarian cancer progression. Thus, the binding of pDox and FABP4 (Fig. 6a) was simulated and calculated the binding affinity by fluorescence polarization (Fig. 6b and 6c). It was indicated that pDox had high binding affinity to FABP4 ($K_d$=23.14 nM), while there was almost no specific binding between Dox and FABP4. In order to build the pDox in FABP4, the structure of linoleic acid was modified and docked it into the binding pocket using the Glide program. Next, the pH and ROS responsive linker chain was built and added onto carbon nine of the lipid chain. The pDox structure was illustrated using Schrodinger Maestro's 3D-sketcher followed by an initial energy minimization procedure, followed by a full-atom, 20 nanosecond molecular dynamics simulation. A weighted binding free energy was -54.58, -81.39, and -80.21 kcal/mol for FABP4 bound lipid, lipid plus the linker, and pDox, respectively. Interestingly, lipid binding affinity was significantly improved after the linker attached. However, the attachment of Dox did not significantly alter the binding affinity, when compared to lipid plus linker. The binding energy of SA-L-Drug varied significantly between different clusters with a high of -45 kcal/mol for cluster 14 and the lowest energy of -98 kcal/mol for cluster 2. The movement of lipid, lipid plus the linker, and pDox were simulated in the FABP4 binding pocket.

74. Next, the cytotoxicity of pDox compared with Dox toward B 16F10 (Fig. 6d), A375 (Fig. 6e), E0771 (Fig. 6f), and MCF-7 (Fig. 6g) cell lines was evaluated. IC50 of pDox to B 16F10, A375, E0771 cells was almost 1.5 times of Dox, but similar toxicity was observed in MCF-7 cell line. Dox and pDox were added to 3T3-L1 cells during their differentiation for drug encapsulation. Then these drug-loaded adipocytes (Dox@adipocytes and pDox@adipocytes) were co-cultured with B 16F10 (Fig. 6h) and E0771 (Fig. 6i) cells in a transwell system. Interestingly, pDox@adipocytes showed more cytotoxicity during the crosstalk between cancer cells and adipocytes compared with Dox@adipocytes. This can be explained by the difference between the route of drug uptake that cancer cells can take up Dox from adipocytes through free diffusion, while pDox in adipocytes can enter cancer cells through FABP4-mediated lipid metabolism pathway with more biocompatibility to cancer cells. The understanding was then tested by adding BMS309403 to the transwell medium to block the lipid transportation from adipocytes to cancer cells. As expected, cytotoxicity of pDox was partially reversed to B16F10 cells (Fig. 6j). Then the lipid amount of Dox and pDox loaded adipocytes was determined. Dox significantly inhibited lipid accumulation in adipocytes. However, lipid accumulation was not significantly suppressed by pDox, indicating improved compatibility of pDox to adipocytes (Fig. 6k). Most pDox was localized in lipid droplets according to confocal microscope imaging (Fig. 6l). During pDox absorption, FABP4 inhibitor did not affect the uptake of pDox in adipocytes. However, it partially inhibited the transportation of pDox from adipocytes to B 16F10 cells in the transwell experiment, which further demonstrated that pDox uptake from adipocytes mainly depended on FABP4 mediated lipid metabolism pathway (Fig. 6m).

75. Next, the potential of RA-loaded adipocytes for delivery of pDox was evaluated. WThe effect of combination therapy was determined using pDox and RA on B16F10 and E0771 cell lines (Fig. 7a and 7b). Treatment with both RA and Dox or pDox significantly enhanced cytotoxicity of Dox and pDox. Then, pDox and RA were simultaneously administrated during the differentiation of 3T3-L1 cells to generate Dox or pDox and RA loaded adipocytes (Dox+RA@adipocytes, pDox+RA@adipocytes). Administration of RA during the differentiation of 3T3-L1 can enhance lipid accumulation in the lipid droplets (Fig. 7c). More pDox can be loaded into RA@adipocytes compared with Dox (Fig. 7d), while RA partially reversed the inhibitory effect of Dox and pDox to lipid droplet formation, leading to enhanced drug loading capacity of adipocytes. This result was further verified by confocal microscope imaging, which showed more lipid droplets formation with more pDox encapsulation in RA@adipocytes (Fig. 7e). The endosome was also labeled, indicating that most pDox localized in lipid droplets. Then pDox +RA@adipocytes or RA@adipocytes were co-cultured with B16F10 cells in transwell. RA@adipocytes displayed promoted pDox uptake in adipocytes and B16F10 cells, which was inhibited by FABP4 inhibitor (Fig. 7f). This transportation of lipid from adipocytes to cancer cells was further confirmed by Western blot. 3T3-L1 cells started to translate FABP4 after the initiation of differentiation. RA@adipocytes or pDox+RA@adipocytes can enhance the amount of FABP4 in B16F10 cells due to lipid transportation, whereas BMS309403 can inhibit this process. Furthermore, there was no significant difference of pDox loading capacity between RA@adipocytes and CLA@adipocytes as well as the pDox uptake of B 16F10 cells in each group. In the same transwell system, pDox+RA@adipocytes had more cytotoxicity to cancer cells compared with RA@adipocytes. This cell killing effect can be partially reversed by FABP4 inhibitor, indicating this process depended on the transportation of FABP4 (Fig. 7g).

76. As discussed above, loss of lipid content in peritumoural TAA caused by tumor cell-triggered lipolysis has been proved to contribute to tumor metastasis by providing energy for tumor cells and inflammatory cytokines to generate a tumor-favored microenvironment. Thus, tumor cell triggered lipolysis can be a new tumor specific metabolism pathway for target drug delivery. To test this, pDox+RA@adipocytes and Dox+RA@adipocytes were co-cultured with B16F10 cells and used mouse fibroblast as a control to compare their drug release profile and lipolysis. B 16F10 significantly triggered release of Dox (Fig. 7h) and pDox (Fig. 7i) from adipocytes, while fibroblast did not affect drug release compared with free adipocytes. Furthermore, B 16F10 induced lipolysis after adipocyte co-culturing for 48 h according to medium free fatty acid concentration, whereas fibroblast did not trigger lipid release from adipocytes (Fig. 7j). Collectively, the release of RA and pDox from adipocytes was mediated by tumor cell promoted lipolysis with FABP4 dependent transportation of pDox and RA during the crosstalk.

77. To validate the therapeutic outcome of pDox+RA@adipocytes *in vivo*, the B16F10 mouse melanoma tumor model was utilized with different treatment intratumourally administrated at day 0 and day 3 when tumor size reached 50-100 mm³. Tumor growth was monitored by measuring individual tumor size (Fig. 8a) and recording the bioluminescence signals of B 16F10 cells. Normally differentiated adipocytes significantly promoted tumor growth, in agreement with previous researches showing that several angiogenesis pathways including JAK/STAT3 and Akt were involved in this process. pDox showed enhanced anti-tumor efficacy when delivered by adipocytes compared with intratumoural injection of free drug, probably because delivery of pDox through lipid metabolism pathway enhanced its biocompatibility to tumor cells. Additionally, Dox showed a slightly better anti-tumor effect when intratumourally injected with RA compared with pDox and RA combination therapy, which was consistent with *in vitro* data (Figs. 6d, 6e, 6f, 6g, 7a, and 7b) showing that free Dox had higher tumor cell killing effects. Each therapeutic group using adipocytes as drug delivery platform showed improved effects, which can be attributed to the role of adipocytes serving as a reservoir for tumor cell-triggered release of Dox, pDox, and RA. Using this delivery vehicle for Dox and RA, the significant antitumour effect was observed with 3/7 tumor inhibition. However, more therapeutic efficacy was obtained from pDox+RA@adipocytes compared with all other groups with 5/7 tumor growth inhibition (Fig. 8a) in one month. Tumor growth (Fig. 8b) was remarkably suppressed in pDox+RA@adipocyte treated group with better survival curves (Fig. 8c) compared with other groups. Intratumoural injection of free drug or drug loaded adipocytes did not affect the body weight of each group. Two days after the second injection of drug or drug loaded adipocytes, tumours were harvested for flow cytometry analysis. Normally differentiated adipocytes can slightly enhance the expression of PD-L1 in tumor cells (Fig. 8d), which was recently reported in prostate cancer cells caused by the activation of JAK/Stat3 pathway and the overexpression of IL-6 and leptin after treatment with adipocyte-conditioned medium. Slightly decreased PD-L1 positive cell population was found in free Dox and RA treated group. Other than the effect of RA, Dox can downregulate cell membrane PD-L1 expression but upregulate its nucleus translocation, which can also contribute to PD-L1 downregulation. Dox+RA@adipocytes showed equal potential for PD-L1 downregulation in tumor compared with pDox+RA@adipocytes. Significant infiltration of CD8⁺ T cells was observed in each combination therapy group, whereas Dox+RA@adipocytes and pDox+RA@adipocytes showed the most promising effects (Fig. 8e). Corresponding to PD-L1 level, Tregs population was significantly decreased under the treatment of Dox+RA@adipocytes or pDox+RA@adipocytes (Fig. 8f). The enhanced Treg population in adipocyte treated group was probably caused by the PPAR-γ mediated recruitment of Tregs from adipose tissue.

78. Residue tumor cells after surgery remain a severe challenge for cancer therapy. Surgery can release the cancer cells from surgical bed or induce the angiogenesis of previously disseminated cancer cells. Recently, Krall *et al.* showed that surgery wounding promoted both local tumor and distant immunogenic tumor growth, indicating the crucial role of the systemic inflammatory response in this process. Inflammatory cytokines, includ-

ing TNF-$\alpha$, IL-6, and CCL2, secreted by TAA can directly induce inflammatory cell accumulation and further establish a low grade inflammation in tumor site. Moreover, elevated circulating concentration of IL-6 were found in obese women, which were associated with the progress of breast cancer. These findings indicate the malignant role of TAA in tumor recurrence process after surgery. Herein, in the tumor resection model (Fig. 9a), tumours grew more rapidly after surgery in adipocytes-treated group compared to control. Using fibrin gel as drug delivery depot, only mice received Dox and RA loaded gel showed more protection from tumor recurrence with delayed tumor growth. pDox@adipocytes showed more efficacy in suppressing tumor growth than gel loading with pDox. Importantly, Dox+RA@adipocytes and pDox+RA@adipocytes significantly protected mice from tumor recurrence with 62.5% and 37.5% recurrence rate, respectively. It was also demonstrated that the tumor uptake of Dox was significantly improved after lipid conjugation in this adipocyte-based delivery depot. Most tumor recurrence can be suppressed for at least two months by pDox+RA@adipocytes with significantly lower tumor volume and higher survival (Fig. 9b and 9c) compared with other groups. With no significant influence on body weight, adipocyte-based drug delivery can be regarded as highly biocompatible with limited toxic effects. One week after surgery, the immune activities were evaluated in tumor microenvironment. RA-treated groups significantly decreased PD-L1 expression in tumor cells, whereas RA@adipocytes showed more promising outcomes (Fig. 9d). As a result, the frequencies for CD8+ T cells were significantly enhanced (Fig. 9e), while a significant decrease of Treg population was observed (Fig. 9f).

79. This work reversed the malignant role of adipocytes associated with tumors and engineered them as a drug delivery trojan horse for RA as an anti-tumor fatty acid and lipid conjugated Dox prodrug for chemotherapy. Significantly enhanced anti-cancer efficacy was achieved by drug transportation through FABP4-mediated lipid metabolism pathway of tumor cells demonstrated in both intratumoural and postsurgical B16F10 melanoma mouse models. Of note, other than the traditional chemotherapy, RA@adipocytes induced an immunogenic tumor phenotype by downregulating PD-L1 expression. This adipocyte-mediated drug delivery strategy can be further extended to treat a variety of diseases associated with lipid metabolism pathway.

**(1) Fluorescent polarization**

80. To determine the binding affinity of Dox or pDox to FABP4, all samples were diluted in PBS buffer. Serial dilutions of FABP4 from 5 to 100 nM were added to 20 nM Dox or pDox. Fluorescence polarization was measured using QuantaMaster 40 UV/VIS Steady State Spectrofluorometer (Photon Technology International). The dissociation constant ($K_d$) was calculated for each by fitting the observed polarization ([mP]) to a general equation for two state binding as previously described.2

**(2) Molecular dynamic simulation**

81. First, a general search of the Protein Data-Bank (PDB) was conducted for the crystal structures of the human FABP4 protein containing small molecule ligands. The X-ray crystal structure of FABP4 bound to linoleic acid was found (PDB: 2Q9S, resolution 2.3Å). The protein structure was optimized using ProteinPrep Wizard with PRIME and EPIK following a procedure used in a previous study. Linoleic acid is the unsaturated analogue of stearic acid (SA), which serves as an anchor for the drug delivery system. Thus, the structure of linoleic acid was modified (by converting double bonds into single bonds), and redocked SA into the binding pocket using the Glide program (SP scoring function) from the Schrodinger software package.

82. Next, the linker (L) chain was manually built and added onto carbon nine of the SA chain. A conformational search was performed using the ConfGen program (OPLS3 force field) to identify a low energy conformer as a starting point. This conformer was further optimized using Hartree Fock geometry minimization with a 6-311G** Pople basis set (this was done due to the large number of rotatable bonds) with Jaguar. The optimized SA-L compound was then docked into the FABP4 binding pocket using induced-fit docking. Induced-fit docking better accounts for protein flexibility by allowing atomic flexibility for both protein and ligand (traditional docking only allows for bond rotation in the ligand while the protein is considered rigid.) This approach successfully identified three stable starting conformations for the SA-L compound. After manual inspection, a conformation was selected that positioned the linker's benzene ring near the protein's surface. This positioning appears clear of side chain residues that would prevent the drug from being attached to the linker.

83. Initially, the same approach used to identify a docked pose of SA-L was applied to create the target molecule, SA-L-Drug. However, the induced-fit docking procedure used for SA-L was unable to generate a stable docked pose of the target SA-L-Drug compound inside the FABP4 binding pocket. Lacking a stable binding pose of SA-L-Drug, the SA-L-Drug structure was manually built using Schrodinger Maestro's 3D-sketcher followed by an initial energy minimization procedure. Importantly, FABP4 surface-exposed residue side chains were visualized to ensure no atomic clashes were created during the construction of the SA-L-Drug compound. In addition, potential for hydrogen bonding networks was considered when placing hydroxyl groups.

84. After the initial SA-L-Drug structure was built in the FABP4 binding pocket, a full-atom, 20 nanosecond molecular dynamics simulation was run using the GPU-accelerated Desmond software (OPLS3 force field, TIP3P water environment, 300K, NTP, 2 fs time step).

Additionally, the FABP4 bound SA and SA-L complexes were subjected to the same simulation. The weighted binding energies for SA, SA-L, and SA-L-Drug were then calculated with MM-GBSA and the Desmond trajectory clustering algorithm. A detailed explanation of this procedure is reported by Hayes et al. All three molecular dynamic simulations were subjected to Schrödinger's Desmond trajectory clustering algorithm. Trajectory clustering creates an RMSD matrix between all frames of a molecular dynamic simulation, then Hierarchical clustering was performed with an average linkage. The clustering groups frames with shared structural orientations together and provides a sampling of all the possible protein and ligand orientations. Importantly, this approach eliminates the possibility of introducing structural sampling bias by only selecting structures (i.e. frames) in a time dependent manner. Frames that are selected by simulation time can share the same 3D-orientations and not accurately represent the true variability or stability in protein and ligand structure. In this study the number of clusters selected was 20 to correlate with the length of the MD simulation, 20ns.

85. Next, the binding free energy was measured for each cluster and a weighted binding free energy was determined for FABP4 bound SA, SA-L, and SA-L-Drug, respectively. The weighted binding free energy was calculated as follows,

$$\Delta G = \sum_i^{20} P_i \Delta G_i$$

86. Where $P_i$ represents the probability of observing cluster i and $\Delta G_i$ is the binding free energy of cluster i. The probability was determined by taking the total number of frames assigned to cluster i and dividing it by the total number of frames in the simulation. Binding energies for each cluster were determined using Schrödinger's Prime MM/GBSA package with a VSGB solvation model. Protein residues within five angstroms of the SA-L-Drug molecule were flexible for the calculation and the remaining protein was treated as rigid. Since an MD analysis had already been performed, it was deemed unnecessary to allow full protein flexibility for the MM/GBSA analysis.

b) Methods

(1) Materials

87. All chemicals were purchased from Sigma-Aldrich and used as received unless specifically explanation. Doxorubicin hydrochloride was purchased from Oakwood Chemical. BMS309403, the FABP4 inhibitor, was purchased from Cayman Chemical. RA (9Z, 11E-CLA) (catalog no. 16413), 10E, 12Z-CLA (catalog no. 04397), and CLA (catalog no. O5507) were purchased from Sigma-Aldrich.

(2) Cell culture

88. Normal cell lines, including 3T3-L1, B16F10, A375, and MCF-7, were purchased from the American Type Culture Collection. E0771 cell line was purchased from CH3 Biosystems. Bioluminescent B16F10 cells (B16F10-luc-GFP) were provided by Dr. Leaf Huang from University of North Carolina at Chapel Hill. B16F10, A375, and MCF-7 cells were cultured in DMEM (Gibco, Invitrogen) with 10% FBS (Invitrogen). E0771 cells were cultured in RPMI 1640 medium with 10% FBS and 10 mM HEPES (Thermo Fisher Scientific). Mouse primary dermal fibroblast was purchased from Cell Biologies (catalog no. C57-6067) and cultured using Fibroblast Medium Kit (catalog no. M2267). For culturing 3T3-L1, DMEM with 10% bovine calf serum (Thermo Fisher Scientific) was used as medium. 3T3-L1 Differentiation Kit (Sigma-Aldrich catalog no. DIF001) was used to differentiated 3T3-L1 preadipocytes. To achieve the maximum loading capacity, 10-20 passages of 3T3-L1 cells were used in this study.

(3) Loading and release of Dox, pDox, and RA

89. For generating RA and Dox or pDox loaded adipocytes, RA (200 μM) and Dox or pDox (500 nM) were added in the maintenance medium (DMEM/F12 (1:1) with 10% FBS and 1.5 mg/mL insulin) and changed for every 48 h. The concentration of RA, Dox, and pDox was optimized to be the maximum concentration that did not significantly cause 3T3-L1 cell death, but can affect lipid accumulation, which was discussed in the main text. Lipid accumulation in adipocytes was evaluated by Oil Red O staining and quantified by optical density measurement at 540 nm. Preadipocytes were cultured, differentiated, and drug encapsulated in 6-well transwell insert, and co-cultured with 5*10$^5$ pre-cultured B16F10 or fibroblasts in 6 well plate to determine drug release profiles, which was calculated according to drug amount remained in adipocytes. Concentration of free fatty acid in co-cultured medium was measured using Free Fatty Acid Quantitation Kit (Sigma-Aldrich, catalog no. MAK044). To measure the amount of Dox and pDox in RA loaded adipocytes, 20 μL Triton X-100 was added to 10$^6$ adipocytes. Then, 100 μL extraction solution (0.75 M HCl in isopropanol) was added and incubated at -20°C overnight. The fluorescence of supernatant at 498(excitation)/591(emission) nm was measured after centrifugation at 20000 g for 15 min. The maintenance medium was changed three to four times for animal work.

(4) Crosstalk between cancer cell and adipocyte

90. Cytotoxicity of drug and fatty acid was determined by MTT assay in 96-well plate after 48 h. Tumor cell killing or promoting effect of drug or fatty acid loaded adipocytes was determined in a transwell system where adipocytes were seeded in the 24 well plate and tumor cells grew in the transwell insert. After culturing for 72 h, cell proliferation of cancer cells in the transwell insert was determined by MTT assay.

91. For Western blot, flow cytometry, and adipokine profiling (R&D Systems catalog no. ARY013), 6 well transwell system was used with cancer cells cultured in the transwell insert and adipocytes in the bottom. Cells or medium were analyzed after co-culturing for 72 h. To determine the role of FABP4 during the crosstalk, 30 $\mu$M BMS309403 was added in the medium to block FABP4. Antibodies used for Western blot included $\beta$-actin (catalog no. sc-47778, Santa Cruz), FABP4 (catalog no. 701158, Thermo Fisher), PD-L1 (catalog no. ab205921, Abeam). PE channel was used to determine pDox fluorescence in adipocytes and cancer cells.

### (5) In vivo tumor studies

92. For subcutaneous model, $1*10^6$ luciferase-tagged B16F10 cells were injected into the right flank of mice. When the tumor reached 50-100 mm$^3$, mice were randomly divided into different groups (n=10-11) with intratumourally injected different formulations on day 0 and day 3, including fibrin gels, pDox loaded fibrin gels, Dox and RA loaded fibrin gels, pDox and RA loaded fibrin gels, normally differentiated adipocytes, pDox loaded adipocytes, Dox and RA loaded adipocytes, and pDox and RA loaded adipocytes. The doses of Dox and pDox were 0.1 and 0.2 mg/kg (usually $7-10*10^6$ adipocytes) since molecular weight of pDox was almost twice of Dox. Tumor size was measured with a digital caliper and monitored by bioluminescence signal using IVIS Lumina imaging system (PerkinElmer) with intraperitoneal injection of luciferin (catalog no. LUCK-100, Gold Biotechnology) at 150 mg/kg. Tumor volume was calculated as long diameter*short diameter$^2$/2.

93. For postsurgical recurrence model, $1*10^6$ luciferase expressed B16F10 cells were subcutaneously injected in the right flank of mice. When tumor size reached 200-300 mm$^3$, most tumor was resected, leaving 1% residual tissue behind. The amount of residual tumor was determined by bioluminescence signal of B16F10 cells before and after surgery. Wound was closed by Autoclip wound clip system. After randomly dividing the mice into different groups (n=10-12), drugs or drug loaded adipocytes were encapsulated into fibrin gels and further implanted into the surgical bed. Tumor growth was monitored by detecting the bioluminescence and measuring tumor size after removing the clips. For both intratumoural and postsurgical models, mice were euthanized when the tumor size exceeded 1.5 cm$^3$.

94. To determine the expression of PD-L1 in tumor cells and the population of T cells, 4 mice were sacrificed in each group to obtain the tumours two days after the second injection of formulation for intratumoural model. For tumor recurrence model, tumours were harvested 1 week after surgery. A single-cell suspension of tumor was prepared using staining buffer (catalog 420201, BioLegend). 20000 events per sample were collected and analyzed using FlowJo software. Antibodies for detecting PD-L1 positive cells, CD8$^+$ T cells, and Tregs included CD3 (catalog 100203, Biolegend), CD4 (catalog 100515, Biolegend), CD8 (catalog 100707, Biolegend), PD-L1 (catalog 124311, Biolegend), FoxP3 (catalog 126403, Biolegend).

### (6) Synthesis of 2,3-dimethylhex-5-ene-2,3-diol.

95. 3-hydroxybutan-2-one (0.44 g, 5 mmol) dissolved in mixed solvent (50 mL, 4:1 THF/H$_2$O) was stirred vigorously, while indium powder (3.3 g, 30 mmol) and then allyl bromide (4 mL, 47 mmol) were introduced. The reaction mixture was stirred at room temperature for three hours, followed by the addition of HCl (3 N, 30 mL) to acquire a clear solution. Then, the mixture was extracted with CHCl$_3$ (2 × 100 mL), concentrated under reduced pressure and passing through a silica column using eluent 1:3 Et$_2$O/PE to give pure product (0.55 g, yield 76%). $^1$H NMR (300 MHz, CDCl$_3$) $\delta$ 5.89 (m, 1H), 5.10 (m, 2H), 2.40 (m, 1H), 2.13 (m, 2H), 2.0 (s, 1H), 1.65 (s, 1H), 1.2 (m, 3H), 1.16 9(s, 3H), 1.10 (s, 3H).

### (7) Synthesis of (4-(4-allyl-4,5,5-trimethyl-1,3,2-dioxaborolan-2-yl)phenyl)methanol.

96. 2,3-dimethylhex-5-ene-2,3-diol (0.21 g, 1.5 mmol), (4-(hydroxymethyl)phenyl)boronic acid (0.2 g, 1.35 mmol), and anhydrous MgSO$_4$ (2 g) were mixed in toluene (50 mL) and refluxed overnight. After filtration, the solvent was removed under reduced pressure and the residual mixture was purified by passing through a silica column (5%-20% EtOAc in PE) to give the product (0.25 g, yield 80%). $^1$H NMR (300 MHz, CDCl$_3$) $\delta$ 7.75 (d, 2H), 7.31 (d, 2H), 5.88 (m, 1H), 5.07 (m, 2H), 4.65 (s, 2H), 2.49 (m, 1H), 2.25 (m, 2H), 1.32 (s, 3H), 1.28 (s, 3H), 1.24 (s, 3H).

### (8) Synthesis of 4-(4-allyl-4,5,5-trimethyl-1,3,2-dioxaborolan-2-yl)benzyl (4-nitrophenyl) carbonate.

97. (4-(4-allyl-4,5,5-trimethyl-1,3,2-dioxaborolan-2-yl)phenyl)methanol (0.2 g) and 4-nitrophenyl carbonochloridate (0.2 g) were dissolved in THF (20 mL) containing Et$_3$N (0.5 mL). After stirred for 4 hours at room temperature, the mixture was concentrated under reduced pressure and passing through a silica column (5% to 20% EtOAc in PE) to give the product (0.2 g, yield 60%). $^1$H NMR (300 MHz, CDCl$_3$) $\delta$ 8.2 (d, 2H), 7.8 (d, 2H), 7.35 (d, 2H), 7.28 (d, 2H), 5.94 (m, 1H), 5.24 (s, 2H), 5.09 (m, 2H), 2.50 (m, 2H), 2.22 (m, 2H), 1.31 (s, 3H), 1.28 (s, 3H), 1.23 (s, 3H).

**(9) Synthesis of 4-(4-(3-((2-mercaptoethyl)thio)propyl)-4,5,5-trimethyl-1,3,2-dioxaborolan-2-yl)benzyl (4-nitrophenyl) carbonate.**

98. 4-(4-allyl-4,5,5-trimethyl-1,3,2-dioxaborolan-2-yl)benzyl (4-nitrophenyl) carbonate (0.2 g, 0.5 mmol), ethane-1,2-dithiol (1 g, 10 mmol) and AIBN (0.2 g, 1.2 mmol) were mixed in toluene (30 mL) and stirred at 40°C while the reaction was monitored by TLC. After reaction was completed, the mixture was concentrated and passing through a silica column (15%-30% EtOAc in PE) to give the product (0.22 g, 90%). $^1$H NMR (300 MHz, CDCl$_3$) $\delta$ 8.21 (d, 2H), 7.8 (d, 2H), 7.35 (d, 2H), 7.29 (d, 2H), 5.24 (s, 2H), 2.66 (m, 4H), 2. 53 (m, 2H), 1.65-1.97 (m, 5H), 1.28 (s, 6H), 1.23 (s, 3H).

**(10) Synthesis of doxorubicin prodrug.**

99. 4-(4-(3-((2-mercaptoethyl)thio)propyl)-4,5,5-trimethyl-1,3,2-dioxaborolan-2-yl)benzyl (4-nitrophenyl) carbonate (30 mg, 0.06 mmol), oleic acid (141.2 mg, 0.5 mmol), and DMPA (4.5 mg, 0.02 mmol) were mixed in THF (50 $\mu$L) under UV irradiation (wavelength of 365 nm) for 30 min. The reaction was monitored by TLC and stopped when all 4-(4-(3-((2-mercaptoethyl)thio)propyl)-4,5,5-trimethyl-1,3,2-dioxaborolan-2-yl)benzyl (4-nitrophenyl) carbonate was reacted, followed by concentration the mixture under reduced pressure. The product was further mixed with doxorubicin hydrochloride (40.6 mg, 0.07 mmol) and Et$_3$N (20 $\mu$L) in DMF (5 mL) overnight in dark. After the reaction was completed, the mixture was concentrated and first purified with large amount of diethyl ether. The crude product was further purified through a silica column (DCM/Me-OH=40:1) to remove most impurity and the purified product was obtained by eluting the column with solvent composed of DCM/MeOH=30:1.

**E. References**

Arab, A., Akbarian, S. A., Ghiyasvand, R. & Miraghajani, M. The effects of conjugated linoleic acids on breast cancer: A systematic review. Adv. Biomed. Res. 5, 115-9175.185573. eCollection 2016 (2016).

Arendt, L. M. et al. Obesity promotes breast cancer by CCL2-mediated macrophage recruitment and angiogenesis. Cancer Res. 73, 6080-6093 (2013).

Au Yeung, C. L. et al. Exosomal transfer of stroma-derived miR21 confers paclitaxel resistance in ovarian cancer cells through targeting APAF1. Nat. Commun. 7, 11150 (2016).

Bachelot, T. et al. Prognostic value of serum levels of interleukin 6 and of serum and plasma levels of vascular endothelial growth factor in hormone-refractory metastatic breast cancer patients. Br. J. Cancer 88, 1721-1726 (2003).

Bae, W. K. et al. Docetaxel-loaded thermoresponsive conjugated linoleic acid-incorporated poloxamer hydrogel for the suppression of peritoneal metastasis of gastric cancer. Biomaterials 34, 1433-1441 (2013).

Behan, J. W. et al. Adipocytes impair leukemia treatment in mice. Cancer Res. 69, 7867-7874 (2009).

Biondo, L. A. et al. Impact of Doxorubicin Treatment on the Physiological Functions of White Adipose Tissue. PLoS One 11, e0151548 (2016).

Buchbinder, E. I. & Hodi, F. S. Melanoma in 2015: Immune-checkpoint blockade - durable cancer control. Nat. Rev. Clin. Oncol. 13, 77-78 (2016).

Cipolletta, D. et al. PPAR-gamma is a major driver of the accumulation and phenotype of adipose tissue Treg cells. Nature 486, 549-553 (2012).

Correa, L. H., Correa, R., Farinasso, C. M., de Sant'Ana Dourado, L. P. & Magalhaes, K. G. Adipocytes and Macrophages Interplay in the Orchestration of Tumor Microenvironment: New Implications in Cancer Progression. Front. Immunol. 8, 1129 (2017).

Demicheli, R., Retsky, M. W., Hrushesky, W. J. & Baum, M. Tumor dormancy and surgery-driven interruption of dormancy in breast cancer: learning from failures. Nat. Clin. Pract. Oncol. 4, 699-710 (2007).

den Hartigh, L. J., Han, C. Y., Wang, S., Omer, M. & Chait, A. 10E,12Z-conjugated linoleic acid impairs adipocyte triglyceride storage by enhancing fatty acid oxidation, lipolysis, and mitochondrial reactive oxygen species. J. Lipid Res. 54, 2964-2978 (2013).

Desai, M. A. et al. An intrinsically disordered region of methyl-CpG binding domain protein 2 (MBD2) recruits the histone deacetylase core of the NuRD complex. Nucleic Acids Res. 43, 3100-3113 (2015).

Dirat, B. et al. Cancer-associated adipocytes exhibit an activated phenotype and contribute to breast cancer invasion. Cancer Res. 71, 2455-2465 (2011).

Elliott, B. E., Tam, S. P., Dexter, D. & Chen, Z. Q. Capacity of adipose tissue to promote growth and metastasis of a murine mammary carcinoma: effect of estrogen and progesterone. Int. J. Cancer 51, 416-424 (1992).

Eschwege, P. et al. Haematogenous dissemination of prostatic epithelial cells during radical prostatectomy. Lancet 346, 1528-1530 (1995).

Farid, R., Day, T., Friesner, R. A. & Pearlstein, R. A. New insights about HERG blockade obtained from protein modeling, potential energy mapping, and docking studies. Bioorg. Med. Chem. 14, 3160-3173 (2006).

Friesner, R. A. et al. Glide: a new approach for rapid, accurate docking and scoring. 1. Method and assessment of docking accuracy. J. Med. Chem. 47, 1739-1749 (2004).

Friesner, R. A. et al. Glide: a new approach for rapid, accurate docking and scoring. 1. Method and assessment of docking accuracy. J. Med. Chem. 47, 1739-1749 (2004).

Fuentes-Mattei, E. et al. Effects of obesity on transcriptomic changes and cancer hallmarks in estrogen receptor-positive breast cancer. J. Natl. Cancer Inst. 106, 10.1093/jnci/dju158. Print 2014 Jul (2014).

Gharpure, K. M. et al. FABP4 as a key determinant of metastatic potential of ovarian cancer. Nat. Commun. 9, 2923-018-04987-y (2018).

Ghebeh, H. et al. Doxorubicin downregulates cell surface B7-H1 expression and upregulates its nuclear expression in breast cancer cells: role of B7-H1 as an anti-apoptotic molecule. Breast Cancer Res. 12, R48 (2010).

Gillilan, R. E., Ayers, S. D. & Noy, N. Structural basis for activation of fatty acid-binding protein 4. J. Mol. Biol. 372, 1246-1260 (2007).

Graves, D. T., Barnhill, R., Galanopoulos, T. & Antoniades, H. N. Expression of monocyte chemotactic protein-1 in human melanoma in vivo. Am. J. Pathol. 140, 9-14 (1992).

Greenwood, J. R., Calkins, D., Sullivan, A. P. & Shelley, J. C. Towards the comprehensive, rapid, and accurate prediction of the favorable tautomeric states of drug-like molecules in aqueous solution. J. Comput. Aided Mol. Des. 24, 591-604 (2010).

Guo, D. D. et al. Synergistic anti-tumor activity of paclitaxel-incorporated conjugated linoleic acid-coupled poloxamer thermosensitive hydrogel in vitro and in vivo. Biomaterials 30, 4777-4785 (2009).

Guo, Z. et al. Probing the alpha-helical structural stability of stapled p53 peptides: molecular dynamics simulations and analysis. Chem. Biol. Drug Des. 75, 348-359 (2010).

Guo, Z. et al. Probing the alpha-helical structural stability of stapled p53 peptides: molecular dynamics simulations and analysis. Chem. Biol. Drug Des. 75, 348-359 (2010).

Halgren, T. A. et al. Glide: a new approach for rapid, accurate docking and scoring. 2. Enrichment factors in database screening. J. Med. Chem. 47, 1750-1759 (2004).

Halgren, T. A. et al. Glide: a new approach for rapid, accurate docking and scoring. 2. Enrichment factors in database screening. J. Med. Chem. 47, 1750-1759 (2004).

Hao, J. et al. Expression of Adipocyte/Macrophage Fatty Acid-Binding Protein in Tumor-Associated Macrophages Promotes Breast Cancer Progression. Cancer Res. 78, 2343-2355 (2018).

Harder, E. et al. OPLS3: A Force Field Providing Broad Coverage of Drug-like Small Molecules and Proteins. J. Chem. Theory Comput. 12, 281-296 (2016).

Hayes, J. M. et al. Kinetics, in silico docking, molecular dynamics, and MM-GBSA binding studies on prototype indirubins, KT5720, and staurosporine as phosphorylase kinase ATP-binding site inhibitors: the role of water molecules examined. Proteins 79, 703-719 (2011).

Howe, L. R., Subbaramaiah, K., Hudis, C. A. & Dannenberg, A. J. Molecular pathways: adipose inflammation as a mediator of obesity-associated cancer. Clin. Cancer Res. 19, 6074-6083 (2013).

Ingram, J. R. et al. PD-L1 is an activation-independent marker of brown adipocytes. Nat. Commun. 8, 647-017-00799-8 (2017).

Jacobson, M. P. et al. A hierarchical approach to all-atom protein loop prediction. Proteins 55, 351-367 (2004).

Jacobson, M. P., Friesner, R. A., Xiang, Z. & Honig, B. On the role of the crystal environment in determining protein side-chain conformations. J. Mol. Biol. 320, 597-608 (2002).

Jorgensen, W. L. & Tirado-Rives, J. The OPLS [optimized potentials for liquid simulations] potential functions for proteins, energy minimizations for crystals of cyclic peptides and crambin. J. Am. Chem. Soc. 110, 1657-1666 (1988).

Krall, J. A. et al. The systemic response to surgery triggers the outgrowth of distant immune-controlled tumours in mouse models of dormancy. Sci. Transl. Med. 10, 10.1126/scitranslmed.aan3464 (2018).

Kwan, H. Y. et al. Subcutaneous adipocytes promote melanoma cell growth by activating the Akt signaling pathway: role of palmitic acid. J. Biol. Chem. 289, 30525-30537 (2014).

Lapeire, L. et al. Cancer-associated adipose tissue promotes breast cancer progression by paracrine oncostatin M and Jak/STAT3 signaling. Cancer Res. 74, 6806-6819 (2014).

Liu, Z. et al. Mature adipocytes in bone marrow protect myeloma cells against chemotherapy through autophagy activation. Oncotarget 6, 34329-34341 (2015).

Liu, Z. et al. Supramolecular stacking of doxorubicin on carbon nanotubes for in vivo cancer therapy. Angew. Chem. Int. Ed Engl. 48, 7668-7672 (2009).

Lu, G. et al. c9, t11- conjugated linoleic acid induces HCC cell apoptosis and correlation with PPAR-gamma signaling pathway. Am. J. Transl. Res. 7, 2752-2763 (2015).

Masso-Welch, P. A. et al. Inhibition of angiogenesis by the cancer chemopreventive agent conjugated linoleic acid. Cancer Res. 62, 4383-4389 (2002).

Miglietta, A. et al. Conjugated linoleic acid induces apoptosis in MDA-MB-231 breast cancer cells through ERK/MAPK signalling and mitochondrial pathway. Cancer Lett. 234, 149-157 (2006).

Nieman, K. M. et al. Adipocytes promote ovarian cancer metastasis and provide energy for rapid tumor growth. Nat. Med. 17, 1498-1503 (2011).

Ochoa, J. J. et al. Conjugated linoleic acids (CLAs) decrease prostate cancer cell proliferation: different molecular mechanisms for cis-9, trans-11 and trans-10, cis-12 isomers. Carcinogenesis 25, 1185-1191 (2004).

Petruzzelli, M. et al. A switch from white to brown fat increases energy expenditure in cancer-associated cachexia. Cell. Metab. 20, 433-447 (2014).

Sakuma, S. et al. cis9, trans11-Conjugated Linoleic Acid Differentiates Mouse 3T3-L1 Preadipocytes into Mature Small Adipocytes through Induction of Peroxisome Proliferator-activated Receptor gamma. J. Clin. Biochem. Nutr. 47, 167-173 (2010).

Santander, A. M. et al. Paracrine Interactions between Adipocytes and Tumor Cells Recruit and Modify Macrophages to the Mammary Tumor Microenvironment: The Role of Obesity and Inflammation in Breast Adipose Tissue. Cancers (Basel) 7, 143-178 (2015).

Sartipy, P. & Loskutoff, D. J. Monocyte chemoattractant protein 1 in obesity and insulin resistance. Proc. Natl. Acad. Sci. U. S. A. 100, 7265-7270 (2003).

Sastry, G. M., Adzhigirey, M., Day, T., Annabhimoju, R. & Sherman, W. Protein and ligand preparation: parameters, protocols, and influence on virtual screening enrichments. J. Comput. Aided Mol. Des. 27, 221-234 (2013).

Shelley, J. C. et al. Epik: a software program for pK( a) prediction and protonation state generation for drug-like molecules. J. Comput. Aided Mol. Des. 21, 681-691 (2007).

Sherman, W., Beard, H. S. & Farid, R. Use of an induced fit receptor structure in virtual screening. Chem. Biol. Drug Des. 67, 83-84 (2006).

Sherman, W., Day, T., Jacobson, M. P., Friesner, R. A. & Farid, R. Novel procedure for modeling ligand/receptor induced fit effects. J. Med. Chem. 49, 534-553 (2006).

Shivakumar, D. et al. Prediction of Absolute Solvation Free Energies using Molecular Dynamics Free Energy Perturbation and the OPLS Force Field. J. Chem. Theory Comput. 6, 1509-1519 (2010).

Shivakumar, D. et al. Prediction of Absolute Solvation Free Energies using Molecular Dynamics Free Energy Perturbation and the OPLS Force Field. J. Chem. Theory Comput. 6, 1509-1519 (2010).

Spencer, M. et al. Adipose tissue macrophages in insulin-resistant subjects are associated with collagen VI and fibrosis and demonstrate alternative activation. Am. J. Physiol. Endocrinol. Metab. 299, E1016-27 (2010).

Stephan, S. B. et al. Biopolymer implants enhance the efficacy of adoptive T-cell therapy. Nat. Biotechnol. 33, 97-101 (2015).

Szatrowski, T. P. & Nathan, C. F. Production of large amounts of hydrogen peroxide by human tumor cells. Cancer Res. 51, 794-798 (1991).

Tanmahasamut, P., Liu, J., Hendry, L. B. & Sidell, N. Conjugated linoleic acid blocks estrogen signaling in human breast cancer cells. J. Nutr. 134, 674-680 (2004).

Van Den Driessche, G. & Fourches, D. Adverse drug reactions triggered by the common HLA-B*57:01 variant: virtual screening of DrugBank using 3D molecular docking. J. Cheminform 10, 3-018-0257-z (2018).

Van Den Driessche, G. & Fourches, D. Adverse drug reactions triggered by the common HLA-B*57:01 variant: a molecular docking study. J. Cheminform 9, 13-017-0202-6. eCollection 2017 (2017).

Wang, C. et al. In situ formed reactive oxygen species-responsive scaffold with gemcitabine and checkpoint inhibitor for combination therapy. Sci. Transl. Med. 10, 10.1126/scitranslmed.aan3682 (2018).

Wang, Y. Y. et al. Mammary adipocytes stimulate breast cancer invasion through metabolic remodeling of tumor cells. JCI Insight 2, e87489 (2017).

Watts, K. S. et al. ConfGen: a conformational search method for efficient generation of bioactive conformers. J. Chem. Inf. Model. 50, 534-546 (2010).

Wen, D., Danquah, M., Chaudhary, A. K. & Mahato, R. I. Small molecules targeting microRNA for cancer therapy: Promises and obstacles. J. Control. Release 219, 237-247 (2015).

Wheate, N. J., Walker, S., Craig, G. E. & Oun, R. The status of platinum anticancer drugs in the clinic and in clinical trials. Dalton Trans. 39, 8113-8127 (2010).

Xu, L. et al. Adipocytes affect castration-resistant prostate cancer cells to develop the resistance to cytotoxic action of NK cells with alterations of PD-L1/NKG2D ligand levels in tumor cells. Prostate 78, 353-364 (2018).

Zeyda, M. & Stulnig, T. M. Adipose tissue macrophages. Immunol. Lett. 112, 61-67 (2007).

## Claims

1. An engineered adipocyte comprising an anti-cancer prodrug and a conjugated fatty acid.

2. The engineered adipocyte of claim 1, wherein the conjugated fatty acid comprises a conjugated linoleic acid isomer 9cis, 11trans, 10trans, and/or 12cis.

3. The engineered adipocyte of claim 1, further comprising a lipid transport protein.

4. The engineered adipocyte of claim 3, wherein the lipid transport protein comprises fatty-acid binding protein 4 (FABP4).

5. The engineered adipocyte of claim 1, wherein the prodrug comprises doxorubicin prodrug

6. The engineered adipocyte of claim 1, wherein the prodrug is conjugated to the conjugated fatty acid via a reactive oxygen species responsive linker.

7. The engineered adipocyte of any of claims 1-6 for use in a method of treating cancer.

8. An engineered adipocyte for use in providing sustained release of an anti-cancer agent to a tumor, wherein the engineered adipocyte is made by the method comprising conjugating the anti-cancer agent to a conjugated fatty acid, and encapsulating the conjugated anti-cancer agent in an adipocyte to make an engineered adipocyte.

## Patentansprüche

1. Ein gentechnisch hergestellter Adipozyt, der ein Anti-Krebs-Prodrug und eine konjugierte Fettsäure umfasst.

2. Der gentechnisch hergestellte Adipozyt nach Anspruch 1, wobei die konjugierte Fettsäure ein konjugiertes Linolsäureisomer 9cis, 11trans, 10trans und/oder 12cis umfasst.

3. Der gentechnisch hergestellte Adipozyt nach Anspruch 1 umfasst ferner ein Lipidtransportprotein.

4. Der gentechnisch hergestellte Adipozyt nach Anspruch 3, wobei das Lipidtransportprotein das Fettsäurebindungsprotein 4 (FABP4) umfasst.

5. Der gentechnisch hergestellte Adipozyt nach Anspruch 1, wobei das Prodrug Doxorubicin-Prodrug umfasst

6. Der gentechnisch hergestellte Adipozyt nach Anspruch 1, wobei das Prodrug über einen auf reaktive Sauerstoffspezies ansprechenden Linker mit der konjugierten Fettsäure konjugiert ist.

7. Der gentechnisch hergestellte Adipozyt nach einem der Ansprüche 1 bis 6 zur Verwendung in einem Verfahren zur Krebsbehandlung.

8. Ein gentechnisch hergestellter Adipozyt zur Verwendung bei der Bereitstellung einer anhaltenden Freisetzung eines Antikrebsmittels an einen Tumor, wobei der gentechnisch hergestellte Adipozyt durch das Verfahren hergestellt wird, das die Konjugation des Antikrebsmittels an eine konjugierte Fettsäure und die Einkapselung des konjugierten Antikrebsmittels in einen Adipozyten zur Herstellung eines gentechnisch hergestellten Adipozyten umfasst.

## Revendications

1. Adipocyte modifié comprenant un promédicament anticancéreux et un acide gras conjugué.

2. Adipocyte modifié selon la revendication 1, dans lequel l'acide gras conjugué comprend un isomère d'acide linoléique conjugué 9cis, 11trans, 10trans et/ou 12cis.

3. Adipocyte modifié selon la revendication 1, comprenant en outre une protéine de transport des lipides.

4. Adipocyte modifié selon la revendication 3, dans lequel la protéine de transport des lipides comprend la protéine 4 de liaison aux acides gras (FABP4).

5. Adipocyte modifié selon la revendication 1, dans lequel le promédicament comprend le promédicament doxorubicine.

6. Adipocyte modifié selon la revendication 1, dans lequel le promédicament est conjugué à l'acide gras conjugué via un lieur sensible aux espèces réactives de l'oxygène.

7. Adipocyte modifié selon l'une quelconque des revendications 1 à 6, destiné à être utilisé dans une méthode de traitement du cancer.

8. Adipocyte modifié destiné à être utilisé pour la fourniture d'une libération prolongée d'un agent anticancéreux vers une tumeur, dans lequel l'adipocyte modifié est

> produit par le procédé comprenant la conjugaison de l'agent anticancéreux
> à un acide gras conjugué, et à l'encapsulation de l'agent anticancéreux conjugué dans un adipocyte pour fabriquer un adipocyte modifié.

FIG. 1A, FIG. 1B, FIG. 1C, FIG. 1D, FIG. 1E, FIG. 1F, FIG. 1G, FIG. 1H, FIG. 1I, FIG. 1J, and FIG. 1K

FIG. 2A, FIG. 2B, FIG. 2C, FIG. 2D, FIG. 2E, FIG. 2F, FIG. 2G, FIG. 2H, FIG. 2I, FIG. 2J, FIG. 2K, and FIG. 2L

FIG. 3A, FIG. 3B, FIG. 3C, FIG. 3D, FIG. 3E, and FIG. 3F

FIG. 4A, FIG. 4B, FIG. 4C, FIG. 4D, FIG. 4E, and FIG. 4F

FIG. 5

FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, and FIG. 6M

FIG. 7A, FIG. 7B, FIG. 7C, FIG. 7D, FIG. 7E, FIG. 7F, FIG. 7G, FIG. 7H, FIG. 7I, and FIG. 7J

FIG. 8A, FIG. 8B, FIG. 8C, FIG. 8D, FIG. 8E, and FIG. 8F

FIG. 9A, FIG. 9B, FIG. 9C, FIG. 9D, FIG. 9E, and FIG. 9F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62754280
- US 3610795 A

**Non-patent literature cited in the description**

- **SENTER et al.** *Bioconjugate Chem.,* 1991, vol. 2, 447-451
- **BAGSHAWE, K.D.** *Br. J. Cancer,* 1989, vol. 60, 275-281
- **BAGSHAWE et al.** *Br. J. Cancer,* 1988, vol. 58, 700-703
- **SENTER et al.** *Bioconjugate Chem.,* 1993, vol. 4, 3-9
- **BATTELLI et al.** *Cancer Immunol. Immunother.,* 1992, vol. 35, 421-425
- **PIETERSZ ; MCKENZIE.** *Immunolog. Reviews,* 1992, vol. 129, 57-80
- **ROFFLER et al.** *Biochem. Pharmacol,* 1991, vol. 42, 2062-2065
- **HUGHES et al.** *Cancer Research,* 1989, vol. 49, 6214-6220
- **LITZINGER ; HUANG.** *Biochimica et Biophysica Acta,* 1992, vol. 1104, 179-187
- **BROWN ; GREENE.** *DNA and Cell Biology,* 1991, vol. 10 (6), 399-409
- Remington: The Science and Practice of Pharmacy. Mack Publishing Company, Easton, 1995
- Handbook of Monoclonal Antibodies. Noges Publications, 1985, 303-357
- **SMITH et al.** Antibodies in Human Diagnosis and Therapy. Raven Press, 1977, 365-389
- **ARAB, A. ; AKBARIAN, S. A. ; GHIYASVAND, R. ; MIRAGHAJANI, M.** The effects of conjugated linoleic acids on breast cancer: A systematic review. *Adv. Biomed. Res.,* 2016, vol. 5
- **ARENDT, L. M. et al.** Obesity promotes breast cancer by CCL2-mediated macrophage recruitment and angiogenesis. *Cancer Res.,* 2013, vol. 73, 6080-6093
- **AU YEUNG, C. L. et al.** Exosomal transfer of stroma-derived miR21 confers paclitaxel resistance in ovarian cancer cells through targeting APAF1. *Nat. Commun.,* 2016, vol. 7, 11150
- **BACHELOT, T. et al.** Prognostic value of serum levels of interleukin 6 and of serum and plasma levels of vascular endothelial growth factor in hormone-refractory metastatic breast cancer patients. *Br. J. Cancer,* 2003, vol. 88, 1721-1726
- **BAE, W. K. et al.** Docetaxel-loaded thermoresponsive conjugated linoleic acid-incorporated poloxamer hydrogel for the suppression of peritoneal metastasis of gastric cancer. *Biomaterials,* 2013, vol. 34, 1433-1441
- **BEHAN, J. W. et al.** Adipocytes impair leukemia treatment in mice. *Cancer Res.,* 2009, vol. 69, 7867-7874
- **BIONDO, L. A. et al.** Impact of Doxorubicin Treatment on the Physiological Functions of White Adipose Tissue. *PLoS One,* 2016, vol. 11, e0151548
- **BUCHBINDER, E. I. ; HODI, F. S.** Melanoma in 2015: Immune-checkpoint blockade - durable cancer control. *Nat. Rev. Clin. Oncol.,* 2016, vol. 13, 77-78
- **CIPOLLETTA, D. et al.** PPAR-gamma is a major driver of the accumulation and phenotype of adipose tissue Treg cells. *Nature,* 2012, vol. 486, 549-553
- **CORREA, L. H. ; CORREA, R. ; FARINASSO, C. M. ; DE SANT'ANA DOURADO, L. P. ; MAGALHAES, K. G.** Adipocytes and Macrophages Interplay in the Orchestration of Tumor Microenvironment: New Implications in Cancer Progression. *Front. Immunol.,* 2017, vol. 8, 1129
- **DEMICHELI, R. ; RETSKY, M. W. ; HRUSHESKY, W. J. ; BAUM, M.** Tumor dormancy and surgery-driven interruption of dormancy in breast cancer: learning from failures. *Nat. Clin. Pract. Oncol.,* 2007, vol. 4, 699-710
- **HARTIGH, L. J. ; HAN, C. Y. ; WANG, S. ; OMER, M. ; CHAIT, A.** 10E,12Z-conjugated linoleic acid impairs adipocyte triglyceride storage by enhancing fatty acid oxidation, lipolysis, and mitochondrial reactive oxygen species. *J. Lipid Res.,* 2013, vol. 54, 2964-2978
- **DESAI, M. A. et al.** An intrinsically disordered region of methyl-CpG binding domain protein 2 (MBD2) recruits the histone deacetylase core of the NuRD complex. *Nucleic Acids Res.,* 2015, vol. 43, 3100-3113

- **DIRAT, B. et al.** Cancer-associated adipocytes exhibit an activated phenotype and contribute to breast cancer invasion. *Cancer Res.,* 2011, vol. 71, 2455-2465
- **ELLIOTT, B. E. ; TAM, S. P. ; DEXTER, D. ; CHEN, Z. Q.** Capacity of adipose tissue to promote growth and metastasis of a murine mammary carcinoma: effect of estrogen and progesterone. *Int. J. Cancer,* 1992, vol. 51, 416-424
- **ESCHWEGE, P. et al.** Haematogenous dissemination of prostatic epithelial cells during radical prostatectomy. *Lancet,* 1995, vol. 346, 1528-1530
- **FARID, R. ; DAY, T. ; FRIESNER, R. A. ; PEARLSTEIN, R. A.** New insights about HERG blockade obtained from protein modeling, potential energy mapping, and docking studies. *Bioorg. Med. Chem.,* 2006, vol. 14, 3160-3173
- **FRIESNER, R. A. et al.** Glide: a new approach for rapid, accurate docking and scoring. 1. Method and assessment of docking accuracy. *J. Med. Chem.,* 2004, vol. 47, 1739-1749
- **FUENTES-MATTEI, E. et al.** Effects of obesity on transcriptomic changes and cancer hallmarks in estrogen receptor-positive breast cancer. *J. Natl. Cancer Inst.,* July 2014, vol. 106
- **GHARPURE, K. M. et al.** FABP4 as a key determinant of metastatic potential of ovarian cancer. *Nat. Commun.,* 2018, vol. 9, 2923-018
- **GHEBEH, H. et al.** Doxorubicin downregulates cell surface B7-H1 expression and upregulates its nuclear expression in breast cancer cells: role of B7-H1 as an anti-apoptotic molecule. *Breast Cancer Res.,* 2010, vol. 12, R48
- **GILLILAN, R. E. ; AYERS, S. D. ; NOY, N.** Structural basis for activation of fatty acid-binding protein 4. *J. Mol. Biol.,* 2007, vol. 372, 1246-1260
- **GRAVES, D. T. ; BARNHILL, R. ; GALANOPOULOS, T. ; ANTONIADES, H. N.** Expression of monocyte chemotactic protein-1 in human melanoma in vivo. *Am. J. Pathol.,* 1992, vol. 140, 9-14
- **GREENWOOD, J. R. ; CALKINS, D. ; SULLIVAN, A. P. ; SHELLEY, J. C.** Towards the comprehensive, rapid, and accurate prediction of the favorable tautomeric states of drug-like molecules in aqueous solution. *J. Comput. Aided Mol. Des.,* 2010, vol. 24, 591-604
- **GUO, D. D. et al.** Synergistic anti-tumor activity of paclitaxel-incorporated conjugated linoleic acid-coupled poloxamer thermosensitive hydrogel in vitro and in vivo. *Biomaterials,* 2009, vol. 30, 4777-4785
- **GUO, Z. et al.** Probing the alpha-helical structural stability of stapled p53 peptides: molecular dynamics simulations and analysis. *Chem. Biol. Drug Des.,* 2010, vol. 75, 348-359
- **HALGREN, T. A. et al.** Glide: a new approach for rapid, accurate docking and scoring. 2. Enrichment factors in database screening. *J. Med. Chem.,* 2004, vol. 47, 1750-1759
- **HAO, J. et al.** Expression of Adipocyte/Macrophage Fatty Acid-Binding Protein in Tumor-Associated Macrophages Promotes Breast Cancer Progression. *Cancer Res.,* 2018, vol. 78, 2343-2355
- **HARDER, E. et al.** OPLS3: A Force Field Providing Broad Coverage of Drug-like Small Molecules and Proteins. *J. Chem. Theory Comput.,* 2016, vol. 12, 281-296
- **HAYES, J. M. et al.** Kinetics, in silico docking, molecular dynamics, and MM-GBSA binding studies on prototype indirubins, KT5720, and staurosporine as phosphorylase kinase ATP-binding site inhibitors: the role of water molecules examined. *Proteins,* 2011, vol. 79, 703-719
- **HOWE, L. R. ; SUBBARAMAIAH, K. ; HUDIS, C. A. ; DANNENBERG, A. J.** Molecular pathways: adipose inflammation as a mediator of obesity-associated cancer. *Clin. Cancer Res.,* 2013, vol. 19, 6074-6083
- **INGRAM, J. R. et al.** PD-L1 is an activation-independent marker of brown adipocytes. *Nat. Commun.,* 2017, vol. 8 (647-017-00799-8
- **JACOBSON, M. P. et al.** A hierarchical approach to all-atom protein loop prediction. *Proteins,* 2004, vol. 55, 351-367
- **JACOBSON, M. P. ; FRIESNER, R. A. ; XIANG, Z. ; HONIG, B.** On the role of the crystal environment in determining protein side-chain conformations. *J. Mol. Biol.,* 2002, vol. 320, 597-608
- **JORGENSEN, W. L. ; TIRADO-RIVES, J.** The OPLS [optimized potentials for liquid simulations] potential functions for proteins, energy minimizations for crystals of cyclic peptides and crambin. *J. Am. Chem. Soc.,* 1988, vol. 110, 1657-1666
- **KRALL, J. A. et al.** The systemic response to surgery triggers the outgrowth of distant immune-controlled tumours in mouse models of dormancy. *Sci. Transl. Med.,* 2018, vol. 10
- **KWAN, H. Y. et al.** Subcutaneous adipocytes promote melanoma cell growth by activating the Akt signaling pathway: role of palmitic acid. *J. Biol. Chem.,* 2014, vol. 289, 30525-30537
- **LAPEIRE, L. et al.** Cancer-associated adipose tissue promotes breast cancer progression by paracrine oncostatin M and Jak/STAT3 signaling. *Cancer Res.,* 2014, vol. 74, 6806-6819
- **LIU, Z. et al.** Mature adipocytes in bone marrow protect myeloma cells against chemotherapy through autophagy activation. *Oncotarget,* 2015, vol. 6, 34329-34341
- **LIU, Z. et al.** Supramolecular stacking of doxorubicin on carbon nanotubes for in vivo cancer therapy. *Angew. Chem. Int. Ed Engl.,* 2009, vol. 48, 7668-7672
- **LU, G. et al.** c9, t11- conjugated linoleic acid induces HCC cell apoptosis and correlation with PPAR-gamma signaling pathway. *Am. J. Transl. Res.,* 2015, vol. 7, 2752-2763

- **MASSO-WELCH, P. A. et al.** Inhibition of angiogenesis by the cancer chemopreventive agent conjugated linoleic acid. *Cancer Res.,* 2002, vol. 62, 4383-4389
- **MIGLIETTA, A. et al.** Conjugated linoleic acid induces apoptosis in MDA-MB-231 breast cancer cells through ERK/MAPK signalling and mitochondrial pathway. *Cancer Lett.,* 2006, vol. 234, 149-157
- **NIEMAN, K. M. et al.** Adipocytes promote ovarian cancer metastasis and provide energy for rapid tumor growth. *Nat. Med.,* 2011, vol. 17, 1498-1503
- **OCHOA, J. J. et al.** Conjugated linoleic acids (CLAs) decrease prostate cancer cell proliferation: different molecular mechanisms for cis-9, trans-11 and trans-10, cis-12 isomers. *Carcinogenesis,* 2004, vol. 25, 1185-1191
- **PETRUZZELLI, M. et al.** A switch from white to brown fat increases energy expenditure in cancer-associated cachexia. *Cell. Metab.,* 2014, vol. 20, 433-447
- **SAKUMA, S. et al.** cis9, trans11-Conjugated Linoleic Acid Differentiates Mouse 3T3-L1 Preadipocytes into Mature Small Adipocytes through Induction of Peroxisome Proliferator-activated Receptor gamma. *J. Clin. Biochem. Nutr.,* 2010, vol. 47, 167-173
- **SANTANDER, A. M. et al.** Paracrine Interactions between Adipocytes and Tumor Cells Recruit and Modify Macrophages to the Mammary Tumor Microenvironment: The Role of Obesity and Inflammation in Breast Adipose Tissue. *Cancers (Basel),* 2015, vol. 7, 143-178
- **SARTIPY, P. ; LOSKUTOFF, D. J.** Monocyte chemoattractant protein 1 in obesity and insulin resistance. *Proc. Natl. Acad. Sci. U. S. A.,* 2003, vol. 100, 7265-7270
- **SASTRY, G. M. ; ADZHIGIREY, M. ; DAY, T. ; ANNABHIMOJU, R. ; SHERMAN, W.** Protein and ligand preparation: parameters, protocols, and influence on virtual screening enrichments. *J. Comput. Aided Mol. Des.,* 2013, vol. 27, 221-234
- **SHELLEY, J. C. et al.** Epik: a software program for pK( a) prediction and protonation state generation for drug-like molecules. *J. Comput. Aided Mol. Des.,* 2007, vol. 21, 681-691
- **SHERMAN, W. ; BEARD, H. S. ; FARID, R.** Use of an induced fit receptor structure in virtual screening. *Chem. Biol. Drug Des.,* 2006, vol. 67, 83-84
- **SHERMAN, W. ; DAY, T. ; JACOBSON, M. P. ; FRIESNER, R. A. ; FARID, R.** Novel procedure for modeling ligand/receptor induced fit effects. *J. Med. Chem.,* 2006, vol. 49, 534-553
- **SHIVAKUMAR, D. et al.** Prediction of Absolute Solvation Free Energies using Molecular Dynamics Free Energy Perturbation and the OPLS Force Field. *J. Chem. Theory Comput.,* 2010, vol. 6, 1509-1519
- **SPENCER, M. et al.** Adipose tissue macrophages in insulin-resistant subjects are associated with collagen VI and fibrosis and demonstrate alternative activation. *Am. J. Physiol. Endocrinol. Metab.,* 2010, vol. 299, E1016-27
- **STEPHAN, S. B. et al.** Biopolymer implants enhance the efficacy of adoptive T-cell therapy. *Nat. Biotechnol.,* 2015, vol. 33, 97-101
- **SZATROWSKI, T. P. ; NATHAN, C. F.** Production of large amounts of hydrogen peroxide by human tumor cells. *Cancer Res.,* 1991, vol. 51, 794-798
- **TANMAHASAMUT, P. ; LIU, J. ; HENDRY, L. B. ; SIDELL, N.** Conjugated linoleic acid blocks estrogen signaling in human breast cancer cells. *J. Nutr.,* 2004, vol. 134, 674-680
- **VAN DEN DRIESSCHE, G. ; FOURCHES, D.** Adverse drug reactions triggered by the common HLA-B*57:01 variant: virtual screening of DrugBank using 3D molecular docking. *J. Cheminform,* 2018, vol. 10 (3-018-0257-z
- **VAN DEN DRIESSCHE, G. ; FOURCHES, D.** Adverse drug reactions triggered by the common HLA-B*57:01 variant: a molecular docking study. *J. Cheminform,* 2017, vol. 9 (13-017-0202-6
- **WANG, C. et al.** In situ formed reactive oxygen species-responsive scaffold with gemcitabine and checkpoint inhibitor for combination therapy. *Sci. Transl. Med.,* 2018, vol. 10
- **WANG, Y. Y. et al.** Mammary adipocytes stimulate breast cancer invasion through metabolic remodeling of tumor cells. *JCI Insight,* 2017, vol. 2, e87489
- **WATTS, K. S. et al.** ConfGen: a conformational search method for efficient generation of bioactive conformers. *J. Chem. Inf. Model.,* 2010, vol. 50, 534-546
- **WEN, D. ; DANQUAH, M. ; CHAUDHARY, A. K. ; MAHATO, R. I.** Small molecules targeting microRNA for cancer therapy: Promises and obstacles. *J. Control. Release,* 2015, vol. 219, 237-247
- **WHEATE, N. J. ; WALKER, S. ; CRAIG, G. E. ; OUN, R.** The status of platinum anticancer drugs in the clinic and in clinical trials. *Dalton Trans.,* 2010, vol. 39, 8113-8127
- **XU, L. et al.** Adipocytes affect castration-resistant prostate cancer cells to develop the resistance to cytotoxic action of NK cells with alterations of PD-L1/NKG2D ligand levels in tumor cells. *Prostate,* 2018, vol. 78, 353-364
- **ZEYDA, M. ; STULNIG, T. M.** Adipose tissue macrophages. *Immunol. Lett.,* 2007, vol. 112, 61-67